# EUROPEAN PATENT APPLICATION

(11) **EP 4 205 655 A1**
(43) Date of publication of application: **05.07.2023**
(21) Application number: 22216481.6
(22) Date of filing: 23.12.2022
(51) Int. Cl.: A61B 6/00

(54) **MEDICAL IMAGE DIAGNOSIS APPARATUS AND SCANNING-RANGE SETTING METHOD**

(30) Priority: 28.12.2021 JP 2021215052
(71) Applicant: Canon Medical Systems Corporation, Tochigi 324-0036 (JP)
(72) Inventor: GATAYAMA, Kazuki, Otawara-shi, 324-0036 (JP)
(74) Representative: Marks & Clerk LLP

(57) **Abstract**

A medical image diagnosis apparatus includes an analyzer unit, an obtainer unit, a setting unit, and an output unit. The analyzer unit obtains a first border line and a second border line that define a scanning range of a scan of a subject to obtain a medical image of the subject, by analyzing a subject image obtained by capturing the subject. The obtainer unit obtains fixed scanning-range information for imaging the subject. The setting unit sets the scanning range based on the fixed scanning-range information and either of the first border line and the second border line obtained by the analyzer unit. The output unit outputs the scanning range set by the setting unit for display on a monitor.

## Description

### FIELD

Embodiments described herein relate generally to a medical image diagnosis apparatus and a scanning-range setting method.

### BACKGROUND

Traditionally, a medical image diagnostic apparatus may allow a selection of an imaging protocol for an examination from imaging protocols contained in a list. The imaging protocols contained in the list are, for example, versatile imaging protocols prepared for particular examinations in compliance with in-hospital rules and regulations or a radiation dose guideline. Further, medical image diagnostic apparatuses are known, which incorporate an automatic scan-planning function that automatically sets, for multiple scans included in the imaging protocol concerned, a scanning range such as an imaging range and a field of view (FOV) according to a camera image or a localizer image of a subject such as a scan image or a volume image.

However, at the time of a follow-up examination, for example, the medical facility that operates such a medical image diagnostic apparatus may desire to apply a different scanning range from the general scanning range in accordance with their policies. Thus, the scanning range set by the automatic scan-planning function may not match with the desirable scanning range according to their operation policies. In such a case it may be necessary to manually correct the automatically set scanning range for scans concerned after the automatic setting. This may cause trouble with setting conditions for each scan, leading to lowering the throughput of image diagnosis using the medical image diagnostic apparatus.

### SUMMARY OF THE INVENTION

According to one aspect of this disclosure, a medical image diagnosis apparatus includes an analyzer unit configured to obtain a first border line and a second border line that define a scanning range of a scan of a subject to obtain a medical image of the subject, by analyzing a subject image obtained by capturing the subject; an obtainer unit configured to obtain fixed scanning-range information for imaging the subject; a setting unit configured to set the scanning range based on the fixed scanning-range information and either of the first border line and the second border line obtained by the analyzer unit; and
an output unit configured to output the scanning range set by the setting unit for display on a monitor.

The above medical image diagnosis apparatus includes a function of determining two boundaries by analysis but performs analysis for only one of the two boundaries intentionally. Thereby, the medical image diagnosis apparatus can exert technical effects of reducing a computational and processing load and a computation time by compensating for the other of the two boundaries with the fixed scanning-range information for imaging the subject.

The obtainer unit may obtain the subject image; the output unit may set, per region to be imaged, at least one border line of a scanning range on a human body image representing a human body model and display, on a monitor, a graphical user interface that allows a designation or a non-designation of a border line of the scanning range based on analysis of the subject image; and an analyzer unit may determine, in response to the designation, at least one border line of the scanning range for a scan of a region to be imaged of the subject by performing the analysis of the subject image with reference to the border line set on the human body image and the human body model. The output unit may display the scanning range based on the determined border line together with the subject image on the monitor.

The human body model may represent a three-dimensional positional relationship among anatomical landmarks in a human body. The analyzer unit may extract anatomical landmarks from the image data; compare the extracted anatomical landmarks and the three-dimensional positional relationship among anatomical landmarks in the human body model, to detect a region to be imaged from the subject image; and determine at least one border line of the scanning range for a scan of the region to be imaged of the subject, based on the detected region to be imaged and the at least one border line of the scanning range on the human body image. The setting unit may set, with reference to the determined border line, the scanning range to be displayed together with the subject image on the monitor.

The at least one border line set on the human body image may include the first border line representing one of a start position and an end position of a scan in the scanning range on the human body image, and the second border line representing the other of the start position and the end position. The apparatus may further include an input unit configured to receive an input to designate only one of the first border line and the second border line. When the set scanning range to be displayed together with the subject image on the monitor is less than a minimum scanning range to be ensured as the scanning range designated by the input, the setting unit may set the scanning range to suffice the minimum scanning range by extending one of the start position and the end position and maintaining the other.

The at least one border line set on the human body image may include the first border line representing one of a start position and an end position of a scan in the scanning range on the human body image, and the second border line representing the other of the start position and the end position. The apparatus may further includes an input unit configured to receive an input to designate either of the first border line and the second border line as being preferentially maintained, in setting the scanning range on the human body image. The setting unit may set the scanning range to be displayed together with the subject image on the monitor such that the scanning range suffices a minimum scanning range to be ensured as the scanning range designated by the input, by maintaining one of the start position and the end position designated by the input and extending the other.

The setting unit may designate a minimum scanning range to be ensured as the scanning range on the human body image, based on previous examination information of the subject. When the set scanning range to be displayed together with the subject image on the monitor is less than the minimum scanning range, the setting unit may set the scanning range to suffice the minimum scanning range by extending one of the start position and the end position and maintaining the other.

The at least one border line set on the human body image may include the first border line representing one of a start position and an end position of a scan in the scanning range on the human body image, and the second border line representing the other of the start position and the end position. The apparatus may further includes an input unit configured to receive an input to designate a center position of the scan in the scanning range on the human body image. The obtainer unit may further obtain fixed scanning-range information as to a scan mode with no couch movement, and the setting unit may set the scanning range to be displayed together with the subject image on the monitor, based on the fixed scanning-range information and one of the first border line and the second border line set according to the center position of the scan.

When the set scanning range to be displayed together with the subject image on the monitor is less than a minimum scanning range to be ensured as the scanning range designated by the input, the setting unit may set the scanning range to suffice the minimum scanning range by extending at least one of the first border line and the second border line and maintaining the center position of the scan.

The setting unit may designate a minimum scanning range to be ensured as the scanning range on the human body image, based on previous examination information of the subject. When the scanning range to be displayed together with the subject image on the monitor is less than the minimum scanning range, the setting unit may set the scanning range to suffice the minimum scanning range by extending at least one of the first border line and the second border line and maintaining the center position of the scan.

The input unit may receive an input to designate the center position of the scan as being preferentially maintained, in setting the scanning range. The setting unit may set the scanning range to suffice the minimum scanning range by extending at least one of the first border line and the second border line and maintaining the center position of the scan.

The at least one border line set on the human body image may include a first border line representing one of a start position and an end position of a scan in the scanning range on the human body image, and a second border line representing the other of the start position and the end position. The first border line and the second border line may define a field of view (FOV) size in the scanning range. The apparatus may further includes an input unit configured to receive an input to designate an FOV size. The setting unit may set the scanning range to be displayed together with the subject image on the monitor, in response to a receipt of the input.

The input unit may receive an input to designate a center position of the FOV. The setting unit may set the scanning range to be displayed together with the subject image on the monitor, in response to a receipt of the input to designate the center position of the FOV.

When the scanning range to be displayed together with the subject image on the monitor is less than a minimum FOV size to be ensured as the FOV size designated by the input, the setting unit may set the scanning range to suffice the minimum FOV size by extending at least one of the first border line and the second border line and maintaining the center position of the FOV.

The input unit may receive an input to designate a minimum FOV size to be ensured as the FOV size designated by the input, based on previous examination information of the subject. When the set scanning range to be displayed together with the subject image on the monitor is less than the minimum FOV size, the setting unit may set the scanning range to suffice the minimum FOV size by extending at least one of the first border line and the second border line and maintaining the center position of the FOV.

The input unit may receive a designation of a maximum magnification factor of the FOV. When the set scanning range to be displayed together with the subject image on the monitor is less than the maximum magnification factor, the setting unit may set the scanning range to suffice the maximum magnification factor by reducing at least one of the first border line and the second border line and maintaining the center position of the FOV.

The apparatus may further include a gantry that performs an X-ray CT scan of the subject. The output unit may output a virtual scanning range simulating the scanning range, to display, on the monitor, the virtual scanning range on an image of the subject captured by a low-dose CT scan by the gantry in a superimposed manner. The input may be directed to the scanning range on display.

The apparatus may further include an optical camera that captures the subject. The output unit may output a virtual scanning range simulating the scanning range, to display, on the display, the virtual scanning range on an image of the subject captured by the optical camera in a superimposed manner. The input may be directed to the scanning range on display.

The output unit may output the determined border line to display the determined border line of the scanning range on the monitor in a highlighted manner.

A scanning-range setting method includes obtaining a first border line and a second border line that define a scanning range of a scan of a subject to obtain a medical image of the subject, by analyzing a subject image obtained by capturing the subject; obtaining fixed scanning-range information for imaging the subject; setting the scanning range based on the fixed scanning-range information and either of the first border line and the second border line obtained by the analyzing; and outputting the scanning range set by the setting for display on a monitor.

### BRIEF DESCRIPTION OF THE DRAWINGS

FIG. 1 illustrates an exemplary structure of an X-ray computed tomography (CT) apparatus according to an embodiment;
FIG. 2 illustrates a first example of a protocol creation screen displayed on a display according to an embodiment;
FIG. 3 illustrates a second example of a protocol creation screen displayed on a display according to an embodiment;
FIG. 4 illustrates a third example of a protocol creation screen displayed on a display according to an embodiment;
FIG. 5 illustrates a fourth example of a protocol creation screen displayed on a display according to an embodiment;
FIG. 6 illustrates an example of a scan screen displayed on a display according to an embodiment;
FIG. 7 is a flowchart illustrating a scanning-range setting process to be performed by an X-ray CT apparatus according to an embodiment;
FIG. 8 illustrates a first example of screen display related to setting of the automatic planning function for the scanning range, to be displayed on a display according to an embodiment;
FIG. 9 illustrates a second example of screen display related to setting of the automatic planning function for the scanning range, to be displayed on a display according to an embodiment;
FIG. 10 illustrates a third example of screen display related to setting of the automatic planning function for the scanning range, to be displayed on a display according to an embodiment;
FIG. 11 illustrates a fourth example of screen display related to setting of the automatic planning function for the scanning range, to be displayed on a display according to an embodiment;
FIG. 12 illustrates a fifth example of screen display related to setting of the automatic planning function for the scanning range, to be displayed on the display according to an embodiment; and
FIG. 13 illustrates a sixth example of screen display related to setting of the automatic planning function for the scanning range, to be displayed on the display according to an embodiment.

### DETAILED DESCRIPTION

According to an embodiment to be described below, a medical image diagnosis apparatus includes an analyzer unit, an obtaining unit, a setting unit, and an output unit. The analyzer unit obtains a first border line and a second border line that define a scanning range of a scan of a subject to obtain a medical image of the subject, by analyzing a subject image obtained by capturing the subject. The obtainer unit obtains fixed scanning-range information for imaging the subject. The setting unit sets the scanning range based on the fixed scanning-range information and either of the first border line and the second border line obtained by the analyzer unit. The output unit outputs the scanning range set by the setting unit for display on a monitor.

Hereinafter, various embodiments of a medical image diagnostic apparatus, a medical image diagnostic system, and a scanning-range setting method will be described with reference to the accompanying drawings. In the following embodiments, parts, portions, components, or, elements denoted by the same reference numerals are considered to perform same or similar functions, and an overlapping explanation thereof will be omitted unless necessary. Further, the same part or portion of an element may be represented in different dimensions or ratios in the drawings. Elements including the same or similar functions may be denoted by reference numerals with ending "a", "b", "c", or "d" for discriminative purpose. In view of better visibility of the drawings, only major elements may be denoted by reference numerals and elements having the same or similar functions may be added with no reference numerals.

### First Embodiment

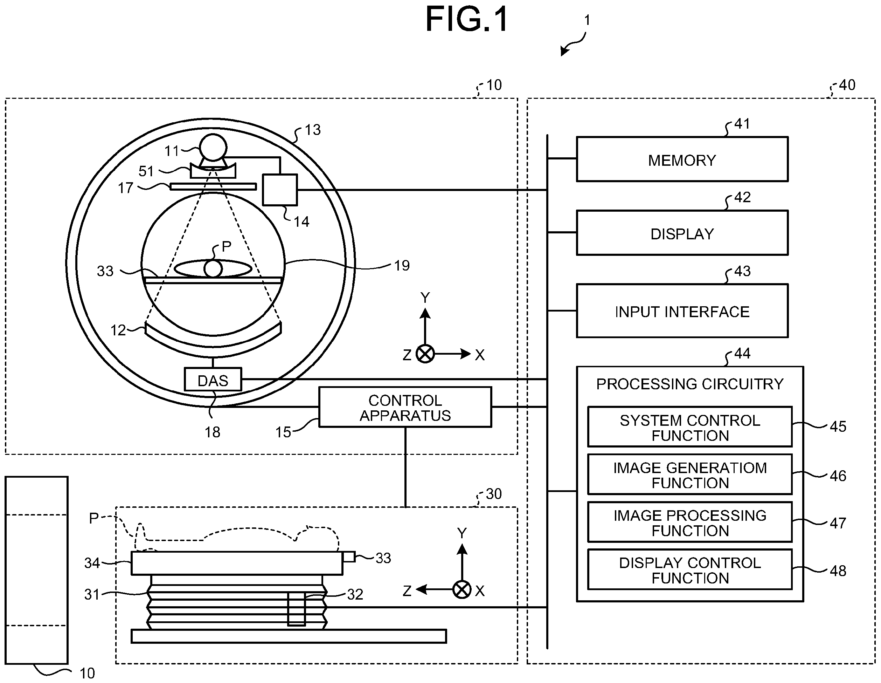

In the present embodiment a medical image diagnostic apparatus that implements an imaging-range setting method is exemplified by an X-ray computed tomography (CT) apparatus. FIG. 1 illustrates an exemplary structure of an X-ray CT apparatus according to an embodiment. The X-ray CT apparatus 1 is configured to irradiate a subject P with an X-ray from an X-ray tube 11 to detect the irradiated X-ray with an X-ray detector 12. The X-ray CT apparatus 1 generates CT image (medical image) data of the subject P based on an output from the X-ray detector 12. The X-ray tube 11 and the X-ray detector 12 are an example of an imaging unit.

As illustrated in FIG. 1, the X-ray CT apparatus 1 includes a gantry 10, a couch 30, and a console 40. FIG. 1 illustrates multiple gantries 10 for illustrative purposes. The gantry 10 is a scanning apparatus including elements for CT scans of the subject P with X-rays. The couch 30 is a carrier apparatus on which the subject P to be CT scanned is laid for localization. The console 40 is a computer that performs control over the gantry 10. For example, the gantry 10 and the couch 30 are installed in a CT examination room while the console 40 is installed in a control room adjacent to the CT examination room. The gantry 10, the couch 30, and the console 40 are communicably connected to one another in a wired or wireless manner. The gantry 10 and the couch 30 are an example of an imaging unit.

The console 40 may not be installed in the control room. For example, the console 40 may be installed together with the gantry 10 and the couch 30 in the same room. Alternatively, the console 40 may be incorporated in the gantry 10.

In the present embodiment, the rotational axis of a rotational frame 13 in a non-tilted state or a longitudinal direction of a table top 33 of the couch 30 is defined as a Z-axis direction. An axial direction orthogonal to the Z-axis direction and horizontal to the floor is defined as an X-axis direction. An axial direction orthogonal to the Z-axis direction and vertical to the floor is defined as a Y-axis direction.

The X-ray CT apparatus 1 is, for example, connected to other apparatuses or devices through in-hospital local area network (LAN) to be able to directly or indirectly communicate with one another. As an example, the X-ray CT apparatus 1 is connected to a picture archiving and communication system (PACS) server that stores and processes medical images, other medical image diagnostic apparatuses, and/or terminal devices to be used by a physician in charge to see images. These apparatuses and devices exchange medical images and other information in compliance with, for example, digital imaging and communications in medicine (DICOM) standard.

In addition, a system including the respective apparatuses and devices as above adopts a hospital information system (HIS) and/or a radiology information system (RIS) to manage various kinds of information. Such a system transmits, for example, examination orders prepared by the terminal devices to the respective medical image diagnostic apparatuses. The individual medical image diagnostic apparatuses directly receive the examination orders prepared by the terminal devices or the PACS server receives the examination orders to create a patient list per modality (modality work list). The individual medical image diagnostic apparatuses obtain patient information from the examination orders or the patient list.

As illustrated in FIG. 1, the gantry 10 includes the X-ray tube 11, the X-ray detector 12, the rotational frame 13, an X-ray high voltage apparatus 14, a control apparatus 15, a wedge 16, a collimator 17, and a data acquisition system (DAS) 18.

The X-ray tube 11 is a vacuum tube having a negative pole (filament) that generates thermoelectrons and a positive pole (target) that generates X-rays by colliding with the thermoelectrons. The X-ray tube 11 is supplied with a high-voltage from the X-ray high-voltage apparatus 14 to irradiate the subject P with X-rays by emitting the thermoelectrons from the negative pole to the positive pole. The X-ray tube 11 is an exemplary X-ray generator unit. By switching the voltage supplied from the X-ray high-voltage apparatus 14 per a predetermined number of views during the X-ray irradiation, dual energy CT scanning can be implemented. In some embodiments, the X-ray CT apparatus 1 may implement only general single energy CT scanning and may not be able to implement dual energy CT scanning. The X-ray CT apparatus 1 may be able to implement multi-energy CT scanning that processes three or more kinds of energy data, other than the dual energy CT scanning.

The X-ray detector 12 detects an X-ray emitted from the X-ray tube 11 and having passed through the subject P and outputs an electric signal corresponding to an amount of the X-ray to the DAS 18. The X-ray detector 12 includes, for example, multiple arrays of detection elements arranged along a single arc about the focal point of the X-ray tube 11 in a channel direction. The X-ray detector 12 has a structure that arrays of detection elements in the channel direction are arrayed in a slice direction (column or row direction), for example. The X-ray detector 12 is an example of an X-ray detector unit.

The X-ray detector 12 is exemplified by an indirect-conversion detector including a grid, a scintillator array, and an optical sensor array. The scintillator array includes multiple scintillators and each scintillator includes a scintillator crystal that outputs an amount of light corresponding to an amount of incident X-rays. The grid is disposed on the X-ray incident side of the scintillator array and includes an X-ray shield plate that functions to absorb scattered X-rays. The grid may be referred to as a collimator (one-dimensional collimator or two-dimensional collimator). The optical sensor array functions to convert an amount of light from the scintillators into an electric signal. The optical sensors may be, for example, photo multipliers (PMT).

The X-ray detector 12 may be a direct-conversion detector including a semiconductor element that converts an incident X-ray into an electric signal.

The rotational frame 13 is an annular frame that supports the X-ray tube 11 and the X-ray detector 12 in opposing positions to rotate the X-ray tube 11 and the X-ray detector 12 under the control of the control apparatus 15 as later described. The rotational frame 13 is provided with an opening 19 with an image field of view (FOV) set. The rotational frame 13 is, for example, a casting made of aluminum. The rotational frame 13 can support the X-ray high voltage apparatus 14, the wedge 16, the collimator 17, and the DAS 18 in addition to the X-ray tube 11 and the X-ray detector 12. The rotational frame 13 can further support other various elements (not illustrated in FIG. 1). The rotational frame 13 is an exemplary rotational unit.

The X-ray high voltage apparatus 14 includes a high voltage generator and an X-ray control device. The high voltage generator includes electric circuitry such as a transformer and a rectifier and functions to generate a high voltage to be applied to the X-ray tube 11 and a filament current to be supplied to the X-ray tube 11. The X-ray control device controls the output voltage in accordance with the X-rays emitted from the X-ray tube 11. The high-voltage generator may be of a transformer type or of an inverter type. Further, the X-ray high-voltage apparatus 14 may be disposed in the rotational frame 13 or in the stationary frame (not illustrated) of the gantry 10. The stationary frame works to rotatably support the rotational frame 13. The X-ray high voltage apparatus 14 is an exemplary X-ray high voltage unit.

The control apparatus 15 includes a driving mechanism such as a motor and an actuator, and processing circuitry including memory and a processor to control the driving mechanism. In accordance with an input signal from an input interface 43 or an input interface included in the gantry 10, the control apparatus 15 controls the operation of the gantry 10 and the couch 30. For example, in response to receipt of input signals, the control apparatus 15 controls the rotation of the rotational frame 13, the tilting of the gantry 10, and the operation of the couch 30.

The control apparatus 15 controls the gantry 10 to tilt by rotating the rotational frame 13 about an axis parallel to the X-axis direction according to tilt-angle information received from an input interface attached to the gantry 10. The control apparatus 15 may be included in the gantry 10 or in the console 40.

The wedge 16 is a filter for adjusting the amount of X-rays emitted from the X-ray tube 11. Specifically, the wedge 16 serves to allow the X-rays emitted from the X-ray tube 11 to transmit therethrough for attenuation, so that the subject P is irradiated with the X-rays from the X-ray tube 11 in a predefined distribution. The wedge 16 is formed of aluminum and has a predetermined target angle and a predetermined thickness. Examples of the wedge 16 include a wedge filter and a bow-tie filter.

The collimator 17 functions to limit the irradiation range of the X-rays having transmitted the wedge 16. The collimator 17 slidably supports lead plates forming slits and adjusts the form of the slits to shield the X-rays. The collimator 17 may be referred to as an X-ray diaphragm.

The DAS 18 functions to read electric signals from the X-ray detector 12. The electric signals correspond to the doses of X-rays detected by the X-ray detector 12. The DAS 18 amplifies the electric signals and integrate or add them in a view period to acquire detection data having a digital value corresponding to the dose of X-rays during the view period. The detection data may be referred to as projection data. The DAS 18 can be implemented by, for example, an application specific integrated circuit (ASIC) incorporating a circuit element that can generate projection data. The DAS 18 transfers the projection data to the console 40 via a non-contact data transfer device, for example. The DAS 18 is an exemplary data obtainer unit.

The detection data generated by the DAS 18 is transmitted by optical communication from a transmitter with light emitting diodes (LED) included in the rotational frame 13 to a receiver with photodiodes included in the non-rotational part (e.g., stationary frame, not illustrated in FIG. 1) of the gantry 10, and then transferred to the console 40. Examples of adoptable data transmission method from the rotational frame 13 to the non-rotational part of the gantry 10 include any non-contact or contact data transfer method in addition to the optical communication.

The couch 30 is a device on which the subject P to be scanned is to be laid and moved, and includes a base 31, a couch driver 32, the table top 33, and a support frame 34. The base 31 is a housing that supports the support frame 34 movably in a vertical direction. The couch driver 32 is a drive mechanism that moves the table top 33 in the longitudinal direction or Z-axis direction, and includes a motor and an actuator. The table top 33 is a plate on which the subject P is to be laid. The table top 33 is placed on the top surface of the support frame 34. The table top 33 can protrude from the couch 30 toward the gantry 10 so as to allow the entire body of the subject P to be scanned. The table top 33 is formed of, for example, carbon fiber reinforced plastic (CFRP) with good X-ray transmissivity and physical property such as rigidity and strength. For example, the table top 33 is hollow inside. The support frame 34 supports the table top 33 movably in the longitudinal direction. The couch 30 is an exemplary medical couch device.

The console 40 includes a memory 41, a display 42, the input interface 43, and processing circuitry 44. The processing circuitry 44, the memory 41, the display 42, and the input interface 43 perform data communications with one another via, for example, a bus. Although the console 40 and the gantry 10 are separately provided herein, the gantry 10 may include the console 40 or part of the elements of the console 40.

The memory 41 can be implemented by, for example, a semiconductor memory device as a random access memory (RAM) or a flash memory, a hard disk, or an optical disk. The memory 41 stores therein, for example, projection data and reconstructed image data. The memory 41 further stores, for example, imaging protocols according to regions or sites to be examined or examination purposes. The memory 41 further stores various programs, for example. The storage regions of the memory 41 may be included in the X-ray CT apparatus 1 or in an external storage device connected via a network. The memory 41 is an exemplary storage unit.

Herein, the imaging protocol is defined for a certain purpose and includes definitions of various conditions and an order of a series of CT scans including at least one or more scans. In the following, the imaging protocol may be referred to as a scan plan or as simply a protocol. Editing the imaging protocol may be referred to as protocol editing.

The display 42 displays various kinds of information. For example, the display 42 outputs medical images (CT images) generated by the processing circuitry 44 and a graphical user interface (GUI) that allows the operator to perform various kinds of operation. The GUI that receives various operations from the operator includes a variety of operation screens that allow scanning-range setting with respect to multiple scans included in an imaging protocol. The display 42 can be any of various kinds of display when appropriate. Examples of the display 42 include a liquid crystal display (LCD), a cathode ray tube (CRT) display, an organic electroluminescence display (OELD), and a plasma display. The display 42 is an exemplary monitor.

The display 42 may be installed in any location in the control room. The display 42 may be included in the gantry 10. The display 42 may be a desktop type or may include a tablet terminal wirelessly communicable with the console 40 itself. The display 42 can be one or two or more projectors.

The input interface 43 serves to receive various inputs from the operator to convert the inputs into electrical signals and output the electrical signals to the processing circuitry 44. As an example, the input interface 43 receives, from the operator, an acquisition condition for acquiring projection data, a reconstruction condition for reconstructing CT images, an image processing condition for generating post-processed images from CT images, and else. The input interface 43 receives the operator's various inputs to the various operation screens for scanning-range setting, for example. The input interface 43 is an exemplary input unit.

Examples of the input interface 43 include a mouse, a keyboard, a trackball, a switch, a button, a joystick, a touch-pad, and a touch panel display, as appropriate. In the present embodiment the input interface 43 is not limited to the one including these physical operational components. Other examples of the input interface 43 include electrical-signal processing circuitry that receives an electrical signal corresponding to an input from an external input device separated from the apparatus to output the electrical signal to the processing circuitry 44. Alternatively, the input interface 43 may be included in the gantry 10. The input interface 43 may include a tablet terminal wirelessly communicable with the console 40 itself.

The processing circuitry 44 controls the X-ray CT apparatus 1 as a whole. The processing circuitry 44 includes, for example, hardware resources including a processor and memory such as a ROM or a RAM. The processing circuitry 44 uses the processor that loads and executes programs on the memory, to implement a system control function 45, an image generation function 46, an image processing function 47, and a display control function 48. The processing circuitry 44 is an exemplary processing unit.

The system control function 45 serves to control the respective functions of the processing circuitry 44 in response to receipt of inputs from the operator via the input interface 43. The processing circuitry 44 implementing the system control function 45 is an example of an obtainer unit, an analyzer unit, and a setting unit.

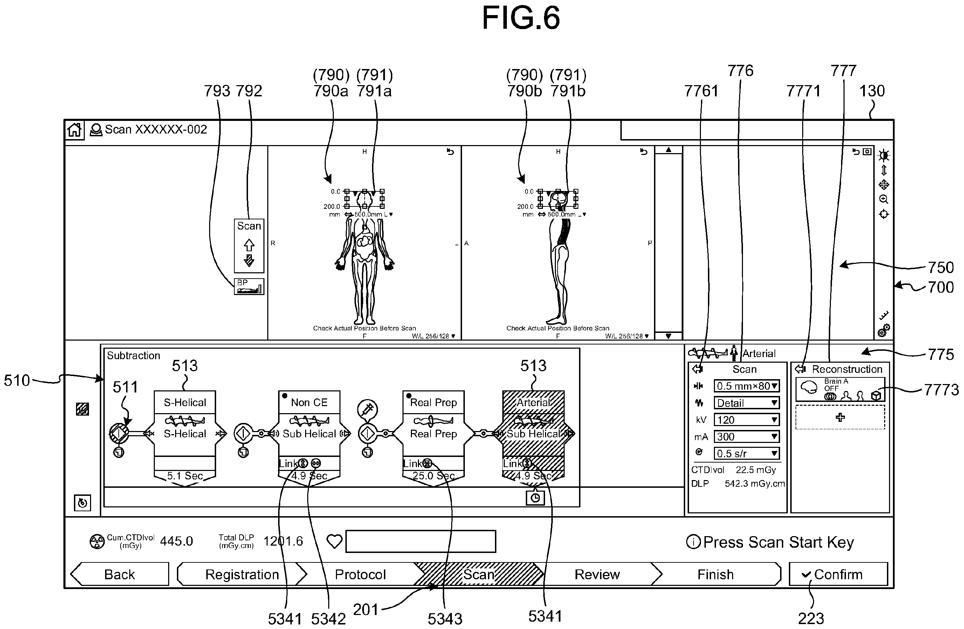

As an example, the processing circuitry 44 controls preparation, editing, or deletion of an imaging protocol preset or an imaging protocol for examination, in accordance with an operator's input received via the input interface 43. The processing circuitry 44 controls, for example, imaging of the subject P in accordance with a selected protocol displayed on a protocol display region of a scan screen (e.g., see FIG. 6).

The processing circuitry 44 uses the image generation function 46 to subject the detection data output from the DAS 18 to pre-processing including logarithm conversion, offset correction, sensitivity correction among the channels, and beam hardening correction, to generate data. The processing circuitry 44 stores the resultant data in the memory 41.
Data before pre-processed (i.e., detection data) and pre-processed data may be collectively referred to as projection data. The processing circuitry 44 perform reconstruction processing to the resultant projection data (pre-processed data) by filtered back projection (FBP), iterative reconstruction, or machine learning to generate CT image data. The processing circuitry 44 stores the CT image data in the memory 41.

The processing circuitry 44 uses the image processing function 47 to convert the CT image data generated by the image generation function 46 into planar image data or three-dimensional image data of any cross section by a known method, in accordance with an operator's input received via the input interface 43. For example, the processing circuitry 44 subjects the CT image data to three-dimensional image processing including volume rendering, surface rendering, intensity projection, multi-planar reconstruction (MPR), and/or curved MPR (CPR) to generate rendering image data in some viewing direction. The image generation function 46 may directly generate three-dimensional image data such as the rendering image data in some viewing direction. The processing circuitry 44 stores the planar image data and the three-dimensional image data in the memory 41.

Further, the processing circuitry 44 uses the image processing function 47 to generate image data for use in display of various display screens that allow scanning-range setting for multiple scans included in the imaging protocol. In the following description, the scanning-range setting for multiple scans included in the imaging protocol may be simply referred to as scanning-range setting.

The processing circuitry 44 uses the display control function 48 to display images on the display 42 based on the various kinds of image data generated by the image processing function 47. The images to be displayed on the display 42 include, for example, CT images based on CT image data, slices based on planar image data of cross-sections, and rendering images based on rendering image data in some viewing direction. The images to be displayed on the display 42 further include images for use in display of the operation screens and images showing notices and warning to the operator. The operation screens include various display screens related to the scanning-range setting. The processing circuitry 44 implementing the display control function 48 can be an exemplary output unit (display control unit). When the processing circuitry 44 is provided outside the medical image diagnostic apparatus, an output interface that outputs an output of the processing circuitry 44 implementing the display control function 48 to the outside may be an exemplary output unit.

The respective functions 45 to 48 may not be implemented by a single piece of processing circuitry. The processing circuitry 44 may be constituted of a combination of multiple independent processors so that the individual processors execute the programs to implement the respective functions 45 to 48. Alternatively, the respective functions 45 to 48 may be implemented by one or two or more processing circuits in a distributed or integrated manner, when appropriate.

The console 40 has been described above as a single console that implements a plurality of functions, by way of example. However, multiple consoles may independently implement a plurality of functions. For example, multiple consoles may include the functions of the processing circuitry 44 such as the image generation function 46 and the image processing function 47 in a distributed manner.

The processing circuitry 44 may be included in an integrated server that collectively processes detection data acquired by multiple medical image diagnostic apparatuses, in addition to in the console 40. Such an integrated server and the medical image diagnostic apparatus of the present embodiment are able to set the scanning ranges of not only multiple medical image diagnostic apparatuses connected via an in-hospital network but also at least one medical image diagnostic apparatus installed outside the hospital such as in an affiliated hospital. Alternatively, a computer as a workstation incorporating the processing circuitry 44 may be provided outside the hospital and shared by multiple medical image diagnostic apparatuses installed at multiple locations such as different hospitals.

The post-processing may be executed by either the console 40 or an external workstation or by both of the console 40 and an external workstation concurrently. Examples of the workstation may include a computer including hardware resources such as a processor that implements the image generation function 46 and the image processing function 47, and memory as a ROM or a RAM.

Although not illustrated in FIG. 1, the X-ray CT apparatus 1 may scan the subject while a contrast is being injected. In such a case a contrast injector and the processing circuitry 44 are communicably connected together so that the X-ray CT apparatus 1 can capture the subject at proper timing in line with the contrast injection timing of the injector.

The X-ray CT image data may be reconstructed by either of a full-scan reconstruction and a half-scan reconstruction. For example, the processing circuitry 44 uses 360-degree projection data of the subject P, i.e., the entire circumference of the subject P in the full-scan reconstruction. The processing circuitry 44 uses projection data of 180 degrees plus a fan angle in the half-scan reconstruction. In the present embodiment the processing circuitry 44 adopts a full-scan reconstruction that reconstructs an image from the entire circumference, 360-degree projection data of the subject P, for simplicity.

The techniques based on the present embodiment are applicable to various types of X-ray CT apparatuses 1 including a third generation CT and a fourth generation CT. The third generation CT refers to a rotate/rotate-type that the X-ray tube and the detector rotate about the subject in an integrated manner. The fourth generation CT refers to a stationary/rotate-type including a large number of X-ray detector elements stationarily arrayed in a ring form that only the X-ray tube rotates about the subject.

The techniques based on the present embodiment are applicable to both a single-source X-ray computed tomography apparatus and a multiple-source X-ray computed tomography apparatus incorporating two or more pairs of X-ray tubes and detectors on the rotational ring.

The present embodiment is described using the X-ray CT apparatus 1 incorporating an integrating X-ray detector 12 as an example. However, the techniques based on the present embodiment can be implemented as an X-ray CT apparatus 1 incorporating a photo-counting X-ray detector.

The X-ray CT apparatus 1 according to the present embodiment can be structured as an upright CT. In such a case the X-ray CT apparatus 1 may include a support that is movable along the rotational axis of the rotational part of the gantry 10 while supporting the subject P in an upright state, in place of the table top 33. Alternatively, the X-ray CT apparatus 1 may exclude the table top 33 or the couch 30. Further, the X-ray CT apparatus 1 according to the present embodiment can be structured as a dental CT or a mobile CT in which the gantry 10 and the couch 30 are movable.

The present embodiment describes, but is not limited to, the X-ray CT apparatus 1 as an example of medical image diagnostic apparatus. The techniques based on the present embodiment are applicable to other types of medical image diagnostic apparatus such as an MRI apparatus, a PET apparatus, a SPECT apparatus, an X-ray diagnostic apparatus, and an ultrasound diagnostic apparatus. In this case, control circuitry included in each medical image diagnostic apparatus can implement the same or similar functions as the processing circuitry 44 of the present embodiment.

An external workstation, a PACS viewer, or a combination thereof may implement various kinds of control related to condition setting in the present embodiment, other than the console 40 of the X-ray CT apparatus 1. Alternatively, a control apparatus may be provided to implement part of the functions of the console 40 for multiple in-hospital medical image diagnostic apparatuses including the X-ray CT apparatus 1 equipped with the gantry 10 and the couch 30. In such a case, for example, the console 40 includes a display 42 that displays screens or GUI images received from the input interface 43 and the control apparatus. The console 40 includes communication circuitry (not illustrated) that transmits inputs from the input interface 43 to the control apparatus via a communication network. The control apparatus processes the inputs and sets scanning ranges in accordance with the inputs, or updates the GUI images in accordance with the inputs. In addition, communication circuitry of the control apparatus transmits the updated GUI images to the communication circuitry of the console 40. In this case the control apparatus implements part of the GUI as described later. The functional allocation between the console 40 and the control apparatus is not limited to this example. The console 40 may handle updating of the GUI images according to the inputs while the control apparatus may handle changing and updating of imaging conditions, scan plans, or protocol information in accordance with the inputs or the updated GUI images. The apparatus that implements the display control related to imaging-protocol editing including setting of reconstruction conditions in an embodiment is an exemplary medical information display control apparatus.

The following will describe in detail scanning-range setting with respect to an imaging protocol for examination in an image diagnosis using the X-ray CT apparatus 1 according to an embodiment, with reference to the accompanying drawings.

Throughout the embodiments, the scanning range refers to an imaging range defining scan start and end positions and a scan size (field of view: FOV).

In an image diagnosis using a medical image diagnostic apparatus such as the X-ray CT apparatus 1, the operator (e.g., a technician or a radiologist) decides items of examination from an examination order sent from a physician in charge to conduct an examination. The operator may select an imaging protocol for the examination concerned from a list containing prepared imaging protocols. The individual imaging protocols contained in the list are prepared as versatile imaging protocols with respect to particular examinations, in compliance with, for example, in-hospital rules and regulations or a radiation dose guideline. The imaging protocol includes, for example, a localizer scan and/or a non-contrast scan or a contrast scan per region or site. Thus, in acquiring X-ray CT image data under a single imaging protocol, the subject undergoes multiple scans in order to acquire X-ray CT image data of the subject per scan.

During an image diagnosis using a medical image diagnostic apparatus such as the X-ray CT apparatus 1, the operator may set the scanning ranges for multiple scans included in the imaging protocol at the time of preparing or editing an imaging protocol for a preset or for examination.

For example, the operator may manually set the scanning range for each of the scans. This manual setting may be, however, troublesome or time-consuming for the operator, so that it is desirable to simplify the scanning-range setting procedure of the operator.

As an example, the medical image diagnostic apparatus including the automatic scan-planning function may automatically set a scanning range for multiple scans included in the imaging protocol concerned as a whole. Herein, the automatic scan-planning function refers to a function to automatically set the scanning range based on image data obtained from various kinds of image, such as a localizer image (scan image or volume image) or a camera image, under an imaging condition set in the protocol. However, the operator may desire to apply a different scanning range from the general scanning range at the time of, for example, a follow-up examination, in compliance with the policies of the medical facility that operates such a medical image diagnostic apparatus. Thus, the scanning range set by the automatic scan-planning function may not match with the scanning range set according to their operation policies. In such a case it may be necessary to manually correct the automatically set scanning range for the scan concerned after the automatic setting. In this regard, it is desired to simplify the scanning-range setting procedure of the operator.

Such troublesome scanning-range setting may result in lowering the throughput of the image diagnosis using the medical image diagnostic apparatus. In view of this, the present embodiment will present the X-ray CT apparatus 1 as an exemplary medical image diagnostic apparatus that can reduce the scanning-range setting procedure of the operator in preparing the imaging protocol, as described below. In other words, the present embodiment will present the X-ray CT apparatus 1 as an exemplary medical image diagnostic apparatus that can offer improvement in throughput of the image diagnosis.

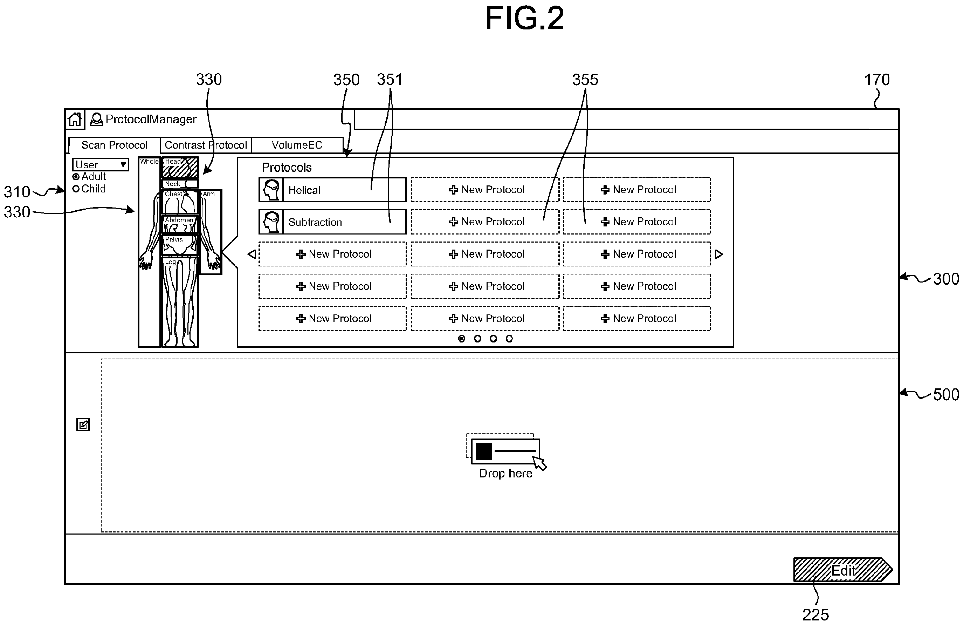

Hereinafter, display screens to be displayed on the display by the processing circuitry 44 will be described with reference to FIG. 2 to FIG. 13. Such display screens allow the user to set examination conditions and else, for example, and may be operable in accordance with inputs from the input interface 43. The items displayed on the screens correspond to examination information and may be changed by a user input. The examination information may be, for example, input, changed, added, or deleted in accordance with the changes made on the screens, as appropriate.

FIG. 2 to FIG. 5 illustrate first to fourth examples of a protocol creation screen 170 to be displayed on the display 42 according to an embodiment.

The protocol creation screen 170 is an operation screen that allows pre-setting of the imaging protocol. Specifically, the protocol creation screen 170 allows creation or correction of an imaging protocol to be selected for the subject P after the information as to the subject P (patient information) is registered.

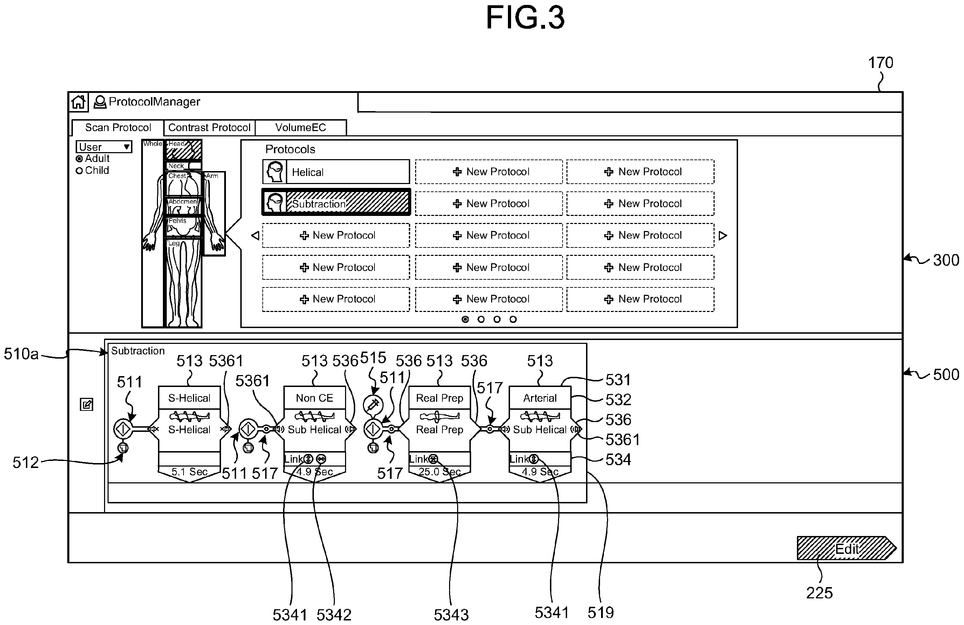

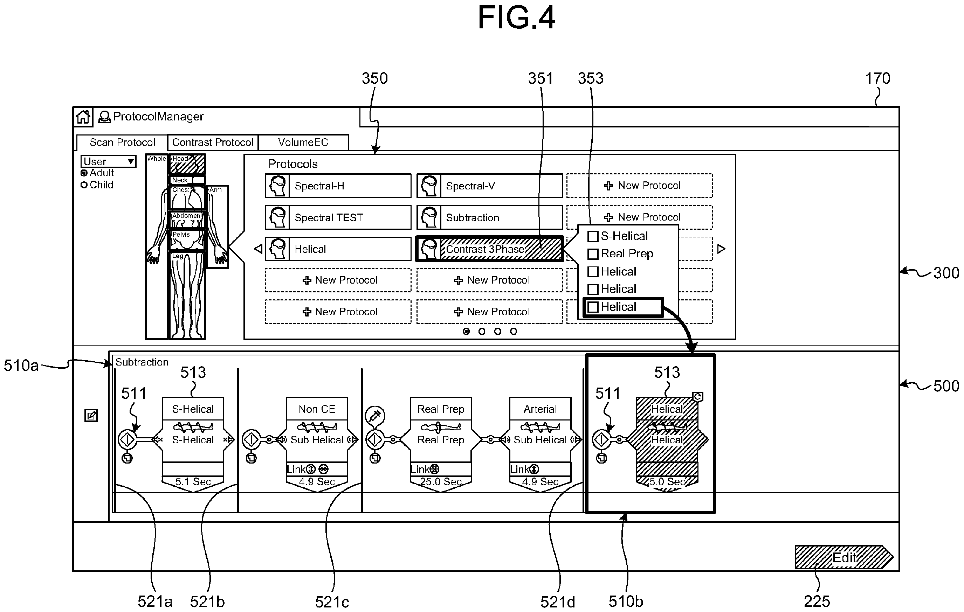

FIG. 2 depicts an initial state of the protocol creation screen 170 by way of example. FIG. 3 and FIG. 4 depict the imaging protocol being displayed on the protocol creation screen 170 by way of example. The processing circuitry 44 displays a protocol selection region 300 and a protocol display region 500 on the protocol creation screens 170 illustrated in FIG. 2 to FIG. 4.

The processing circuitry 44 displays, for example, an attribute selector 310 for setting a patient attribute, such as "Adult" and "Child", on the protocol selection region 300 as illustrated in FIG. 2. The processing circuitry 44 further displays, for example, a region selector 330 for selecting a region to be examined such as "Whole", "Head", "Neck", "Chest", "Abdomen", "Pelvis", "Leg", and "Arm" as illustrated in FIG. 2. The processing circuitry 44 displays a patient attribute and a region to be examined as selected in a highlighted manner.

The processing circuitry 44 displays, for example, an imaging protocol list on a list display region 350 of the protocol selection region 300, as illustrated in FIG. 2. The imaging protocol list contains two or more preset imaging protocols applicable to the region selected using the region selector 330. Hereinafter, the imaging protocol list may be simply referred to a protocol list. The processing circuitry 44 displays, for example, icons 351 on the list display region 350 as illustrated in FIG. 2. The icons 351 correspond to at least one valid, preset imaging protocol with respect to the patient attribute and the region to be examined as set. The "Helical" icon 351 represents a protocol for performing a typical helical CT scan. The "Subtraction" icon 351 represents a protocol for obtaining a differential image between images resulting from two scans before and after a contrast injection. Hereinafter, the imaging protocol represented by the icon 351 on the display may be simply referred to as an imaging protocol for the sake of simplicity. Also, the imaging protocol being imaging information associated with the icon 351 may be simply referred to as the icon 351.

The processing circuitry 44 further displays, for example, icons 355 on the list display region 350 of the protocol selection region 300 as illustrated in FIG. 2. The icons 355 represent new protocols. As an example, the operator selects one of the icons 355 representing new protocols to start preparing a protocol. In response to the operator's selection of the icon 355 representing a new protocol, the processing circuitry 44 determines a display position of the icon 351 representing an imaging protocol which is created by the subsequent processing, for example.

As an example, the operator can select one of the icons 355 representing new protocols displayed on the list display region 350, and drag and drop one icon representing an imaging protocol of interest among the icons 351 representing the preset imaging protocols, onto the protocol display region 500 of the protocol creation screen 170. The drag and drop operation may be implemented with a mouse or a touch panel.

In response to a receipt of the operator's input via the input interface 43, the processing circuitry 44 displays the icon 351 representing the selected imaging protocol on the protocol selection region 300 in a highlighted manner, to show that the icon 351 is being in an active state. The processing circuitry 44 further displays, on the protocol display region 500, a selected protocol icon 510a (icon 510) representing the imaging protocol indicated by the selected icon 351.

The operator may select any of the imaging protocol icons 351 on the protocol selection region 300 to display the imaging protocol icon on the protocol display region 500 by any operational method, in addition to the drag and drop operation. As an example, the operator may single-click the icon 351 representing an imaging protocol of interest among the icons displayed on the protocol selection region 300 of the protocol creation screen 170. The operator may then single-click the protocol display region 500 while the imaging protocol icon 351 of interest is being highlighted. In response to a receipt of the operator's input via the input interface 43, the processing circuitry 44 may further display the single-clicked imaging protocol icon 351 in an active state on the protocol selection region 300. Upon the protocol display region 500 being clicked, the processing circuitry 44 may display detailed information as to the imaging protocol icon 351 being in an active state, i.e., the selected protocol icon 510, on the clicked protocol display region 500.

The processing circuitry 44 displays, for example, the icon 510 (icon 510a) representing detailed information of the selected imaging protocol on the protocol display region 500, as illustrated in FIG. 3. Hereinafter, the selected imaging protocol may be simply referred to as a selected protocol. As the icon 510 representing detailed information of the selected imaging protocol, the processing circuitry 44 displays, in time series or in order of execution, pieces of information representing elements of the selected protocol including a scan and an operator's manipulation, for example, from left to right on the screen as illustrated in FIG. 3.

In connection with the imaging protocol represented by the "Subtraction" icon 351, the processing circuitry 44 displays, for example, irradiation icons 511 representing turning-ON of an irradiation switch and an icon 515 representing a contrast injection as information representing the operator's manipulations, as illustrated in FIG. 3. Start mode icons 512 for setting a start mode are displayed below the irradiation icons 511. Setting a start mode refers to setting a location where a scan is started by pressing the irradiation switch. The location where the irradiation switch is pressed is selected from among, for example, a control pad, the gantry, and a manual switch.

Moreover, the processing circuitry 44 displays, for example, scan icons 513 as information on scans included in the selected protocol, as illustrated in FIG. 3. The scan icons 513 represent scans as "S-Helical", "Non CE", "Real Prep (i.e., CT fluorography for monitoring a contrast", and "Arterial (i.e., arterial phase scan)" included in the imaging protocol represented by the subtraction icon 351. As such, each imaging protocol includes at least one scan.

The individual scan icons 513 show the scan information in the form of certain text or image information. In FIG. 3, for example, each scan icon has a substantially rectangular frame 531 including a top region 532 showing the name of a scan and a middle region 536 below the top region showing a scan type by text and an icon. FIG. 3 illustrates scan types with text and icons such as "S-Helical (localizer scan or scanogram by helical scan)", "Sub-Helical (helical scan by subtraction)", and "Reap-Prep" in the region 5361, by way of example. In a region 534 below the middle region 536, displayed are the text "Link" representing that conditions for different scans are to be synchronized and an icon representing a type of condition to be synchronized. For example, the "Non CE" scan icon 513 includes an icon 5341 representing a linkage between Z-directional ranges (imaging ranges) in a scanning range or a reconstruction range and an icon 5342 representing a linkage between FOV sizes in a scanning range or a reconstruction range. "S-Helical" is a scanogram, therefore, there is no display of "Link" in the scan frame, showing no linkage between imaging conditions. In the "Arterial" scan frame, "Link" is displayed to show a linkage between imaging conditions, together with an icon 5341 representing Z-directional imaging ranges to be linked. In the "Real Prep" scan frame, an icon 5343 is displayed next to "Link". The icon 5343 represents a mode for performing a subsequent scan (in this example "Arterial" scan) in a particular condition so that the scan can be started as early as possible after the "Prep".

In a bottom region of each frame, a scan time required is displayed.

The substantially rectangular frame 531 is provided with triangular protrusions 536 on the right and left sides, on which speaker icons 5361 are displayed. Different types of the speaker icons 5361 show issuance or non-issuance of voice conveying a scan start (left side) and a scan end (right side).

Furthermore, the processing circuitry 44 displays, for example, a connection icon 517 between elements to be successively performed among the elements included in the selected protocol icon 510, as illustrated in FIG. 3. The processing circuitry 44 also displays an icon 519 indicating the area of the selected protocol. In FIG. 3 the icon 51 is in the form of a rectangular frame as an example. The icon 519 indicating the area of the selected protocol may not be displayed.

The scan icons 513, the irradiation icons 511, the start mode icons 512, the connection icons 517, and the various icons displayed in the scan frames are changeable in accordance with user inputs to the respective icons. Various kinds of conditions for the protocols can be changed in accordance with the user inputs. For example, by clicking the connection icon 517 in the "Real Prep" scan icon, the "Real Prep" scan and the "Non CE" scan can be connected on display (same as the display connecting between "Real Prep" and "Arterial"). By pressing the irradiation switch at the time of a start of the "Non CE" scan, the connected scans can be performed automatically as appropriate. In the connected scans, a succeeding-scan start timing can be set as an elapsed time from a preceding event. The succeeding-scan start timing can be set, for example, by setting an elapsed time from the irradiation switch ON timing, "Non CE" scan start or end timing, or else. Such setting can be made in accordance with a user input to the protocol display region 500.

With reference to FIG. 2 and FIG. 3, a description has been made on an example of displaying the selected protocol icon 510 on the protocol creation screen 170 by a drag and drop operation to allow the operator to see the details of the imaging protocol, however, it is not construed as limiting. As another example, the details of an imaging protocol represented by an icon 351 can be displayed in the following manner.

Referring to FIG. 4, the processing circuitry 44 displays a scan list 353 on the protocol selection region 300. The scan list 353 shows scans included in an imaging protocol selected by the operator from the list display region 350. The processing circuitry 44 displays, for example, the scan list 353 including icons of scans included in an imaging protocol represented by a "Contrast 3Phase" icon 351 as illustrated in FIG. 4. In the example of FIG. 4, the scan list 353 contains "S-Helical", "Real Prep", "Helical", "Helical", and "Helical" scan icons.

As an example, the operator single-clicks the highlighted icon 351 representing the imaging protocol of interest again. In response to a receipt of the operator's input via the input interface 43, the processing circuitry 44 pops up the scan list 353 for the imaging protocol represented by the clicked, active icon 351 on display. Through the same or similar operation to the icon 351 on the list display region 350, the operator can select a scan from the scans included in the imaging protocol represented by the icon 351, i.e., the scans in the scan list 353. The processing of the processing circuitry 44 is also same or similar thereto.

The pop-up display of the scan list 353 may show only the scan names as illustrated in FIG. 4 or may be of the same or similar display form as that on the protocol display region 500 in FIG. 2 and FIG. 3.

In this manner the operator can simply check the imaging protocol of interest on the pop-up display of the scan list 353 by single-clicking the icon 351 concerned twice on the list display region 350.

During the display of the scan list 353 showing detailed protocol information, another imaging protocol can be added, inserted, replaced, or deleted. An example of additionally selecting the icon 351 representing a "Contrast 3Phase" imaging protocol on the protocol creation screen 170 of FIG. 3 will be described below with reference to FIG. 4.

As an example, the operator drags and drops the "Contrast 3Phase" protocol icon 351 onto the protocol display region 500. Upon detecting a start of the drag and drop operation to the icon 351 from the operator's input via the input interface 43, the processing circuitry 44 displays, on the protocol display region 500, icons 521 (521a to 521d) indicating insertable positions of the icon 510 representing detailed information of the imaging protocol. In FIG. 4 the icons 521 are depicted as solid lines by way of example. Thereby, the operator can easily know the insertable positions of the icon 510 associated with the "Contrast 3Phase" imaging protocol, at the time of starting the drag and drop operation.

For example, the operator drops the icon 351 of "Contrast 3Phase" imaging protocol in an area behind the protocol display region 500 (icon 521d and the right side thereof in FIG. 4). The processing circuitry 44 then additionally displays a "Contrast 3Phase" icon 510b behind or at the end of the "Subtraction" icon 510a, as illustrated in FIG. 4.

The operator can also drop the "Contrast 3Phase" icon 351 in the upper space of the protocol display region 500. The processing circuitry 44 then replaces the "Subtraction" icon 510a on display with the "Contrast 3Phase" icon 510b.

The operator can drag and drop the "Subtraction" icon 510a being displayed on the protocol display region 500 at the end of the protocol creation screen 170 or on the list display region 350. The processing circuitry 44 then deletes the "Subtraction" icon 510a from the protocol display region 500. In addition the processing circuitry 44 can separately display an icon representing a drop position on the protocol creation screen 170, to delete the selected protocol icon 510 from the protocol display region 500.

With respect to a combination of imaging protocols, the operator can drag and drop, for example, the selected protocol icons 510 (icons 510a, 510b in FIG. 4) displayed on the protocol display region 500, to rearrange the icons on a protocol basis. The processing circuitry 44 displays the icons 521 (521a to 521d) representing the insertable positions of the icon 510 associated with the imaging protocol on the protocol display region 500, upon detecting a start of the drag and drop operation to one of the icons 510 on the protocol display region 500 from the operator's input via the input interface 43, as described above.

With reference to FIG. 4, a description has been made on examples of the protocol-basis protocol editing including, but not limited to, an addition or insertion of the imaging protocol with respect to the protocol display region 500, and a replacement, a transfer or rearrangement, or a deletion of the selected protocol on display. For example, a scan or scans can be added or inserted to the scans included in the imaging protocol on the protocol display region 500 by a drag and drop operation. Likewise, the respective scan icons included in the icons 510 (510a, 510b) displayed on the protocol display region 500 can be replaced, transferred or rearranged, or deleted on a scan basis.

In accordance with an operator's input, the processing circuitry 44 may release the connection between the scans to be successively executed among the scan icons 513, for example, as indicated by the connection icon 517 illustrated in FIG. 3. In this case the operator can insert a protocol or scan icon to the position of the connection icon 517.

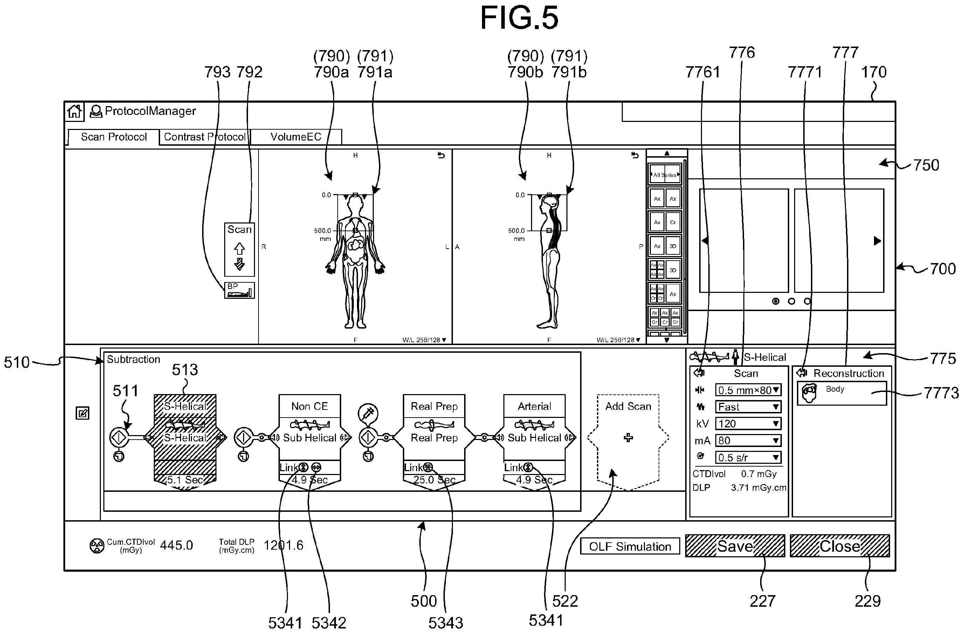

The protocol creation screens 170 of FIG. 3 and FIG. 4 transition to the protocol creation screen 170 of FIG. 5, in response to an operator's selection of an edit button 225.

FIG. 5 illustrates the protocol creation screen 170 after the protocol is loaded. The processing circuitry 44 displays the protocol display region 500 and a scan information display region 700 on the protocol creation screen 170 of FIG. 5.

In response to an operator's selection of the edit button 225 while the selected protocol icon 510 is being displayed on the protocol display region 500, the processing circuitry 44 loads the selected protocol indicated by the icon 510 displayed on the protocol display region 500, as illustrated in FIG. 5.

The operator can now edit the imaging protocol on the protocol creation screen 170 of FIG. 5, such as addition, insertion, replacement, or deletion of the imaging protocol icon 351. In addition, the processing circuitry 44 may display an icon 522 indicating an insertable position of the scan icon 513. The icon 521 may be displayed in place of the icon 522. The icon 522 may be displayed on the protocol creation screens 170 of FIG. 3 and FIG. 4.

The processing circuitry 44 displays, for example, information of the scanning ranges 791 (791a, 791b), scan direction information 792, and body position information (BP) 793 on a human body image 790 such as a human body model or a subject image in an imaging information display region 750, as illustrated in FIG. 5. Further, the processing circuitry 44 displays an image obtained from a scanogram (localizer scan) on the scan information display region 700, which allows the operator to set the scanning range. During or after a scan, the processing circuitry 44 displays an image obtained by the scan on a scan screen 130 as described later, which allows the user to see the image. In addition the processing circuitry 44 may display an image of the subject P captured by an optical camera on the imaging information display region 750.

The processing circuitry 44 also displays, for example, various kinds of scan information including scan condition information 776 and reconstruction condition information 777 on a detailed condition display region 775 of the imaging information display region 750, as illustrated in FIG. 5. In the example of FIG. 5, the scan condition information 776 includes tabs representing the respective conditions as scanning range "0.5 mm x 80", scan speed "Fast", tube voltage [kV] "120", tube current [mA] "80", and rotation speed "0.5 s/r". In the example of FIG. 5, the scan condition information 776 also includes display of computed tomography dose index (CTDI) vol. "0.7 mGy" and dose length product (DLP) "3.71 mGy.cm". In the example of FIG. 5, the reconstruction condition information 7773 includes a "Body" icon representing a body condition of the subject.

The processing circuitry 44 further displays, for example, an expand icon 7761 on the scan condition information 776 on display, as illustrated in FIG. 5. The expand icon 7761 represents a detailed display of the scan condition. In response to an operator's selection of the expand icon 7761, the processing circuitry 44 displays detailed scan condition information as to the scan represented by the selected scan icon 513. Likewise, as illustrated in FIG. 5, the processing circuitry 44 displays, for example, an expand icon 7771 on the reconstruction condition information 777 on display. The expand icon 7771 represents a detailed display of the reconstruction condition. In response to an operator's selection of the expand icon 7771, the processing circuitry 44 displays detailed reconstruction condition information as to the scan represented by the selected scan icon 513.

The operator selects a save button 227 to register, as an imaging protocol preset to be displayed as the icons 351, a series of selected protocols represented by the icon 510 displayed on the protocol display region 500 of the protocol creation screen 170 in FIG. 5. In response to the selection of the save button 227, the processing circuitry 44 registers, as an imaging protocol preset, the series of selected protocols represented by the icon 510 displayed on the protocol display region 500. In addition the operator selects a close button 229 to end the protocol creation. In response to the selection of the close button 229, the processing circuitry 44 ends the display of the protocol creation screen 170.

The protocol display region 500 may be displayed on a protocol edit screen and the scan screen 130 (FIG. 6) in addition to on the protocol creation screen 170 for presetting imaging protocols.

The protocol edit screen refers to an operation screen for selecting, editing, and adjusting the protocol to be used in examination. The protocol edit screen includes the protocol display region 500 which shows the details of the protocol in the same manner as the protocol creation screen 170 or the scan screen 130. The protocol edit screen may be also referred to as a protocol adjust screen.

Detailed conditions for the scans included in the imaging protocol are mainly adjusted in the phase subsequent to the protocol editing or adjustment, i.e., on the scan screen 130 (FIG. 6). The protocol edit screen is mainly used for setting the whole procedure of the protocol such as the relationship between or among the scans, irradiation switch ON timing, and contrast injection timing. The protocol edit screen is further used to make settings as to the progress of a scan such as issuance or non-issuance of audio guidance before and after each scan and to make minor settings as to a body position, e.g., a head first or a feet first, which are unlikely to be changed through the entire protocol. Regarding the body position, the term "a head first" refers to a body position that the subject is inserted into the gantry 10 from the head side. The term "a feet first" refers to a body position that the subject is inserted into the gantry 10 from the feet side. The imaging protocols are generally associated with regions or sites, therefore, the region of interest is to be selected on the protocol edit screen.

FIG. 6 illustrates an exemplary scan screen 130 to be displayed on the display 42 according to an embodiment. The scan screen 130 is an operation screen for conducting an examination by a series of scans included in the imaging protocol which has been selected, edited, and/or adjusted on the protocol edit screen.

As an example, the operator can shift the screen to the scan screen 130 while maintaining the display of the protocol display region 500, by manipulating a "Next" button on the protocol edit screen, for example. In response to a receipt of the operator's input via the input interface 43, the processing circuitry 44 loads information to be displayed on the scan information display region 700, such as a scan condition. The processing circuitry 44 then shifts the protocol edit screen to the scan screen 130.

As with on the protocol creation screen 170 of FIG. 5, the processing circuitry 44 displays, on the scan screen 130, the scan information display region 700 including the imaging information display region 750 and the detailed condition display region 775.

As illustrated in FIG. 6, the processing circuitry 44 further displays, on the scan screen 130, the protocol display region 500 used for the protocol setting on the protocol edit screen in the previous protocol selection phase. In other words, the multiple scan icons 513 displayed on the protocol display region 500 of the scan screen 130 represent multiple scans to be performed to the subject P.

Further, the processing circuitry 44 performs control of the multiple scans as indicated by the scan icons 513 on the protocol display region 500, in the set scanning range on the scan screen 130. For example, in response to an operator's selection of a "Confirm" button, the processing circuitry 44 performs a scan ("Arterial" scan in the example of FIG. 6) highlighted on the display. Specifically, the processing circuitry 44 controls the scans included in the imaging protocol in the set scanning range according to the CT scan control method of an embodiment.

The processing circuitry 44 further displays, for example, a "Scan" button in a highlighted manner on the scan screen 130. The "Scan" button indicates a scan phase of an examination procedure. The scan screen 130 displays the edited protocol together with a detailed scan condition designated from the protocol. The scan screen 130 also displays a scan image, allowing the operator to see.

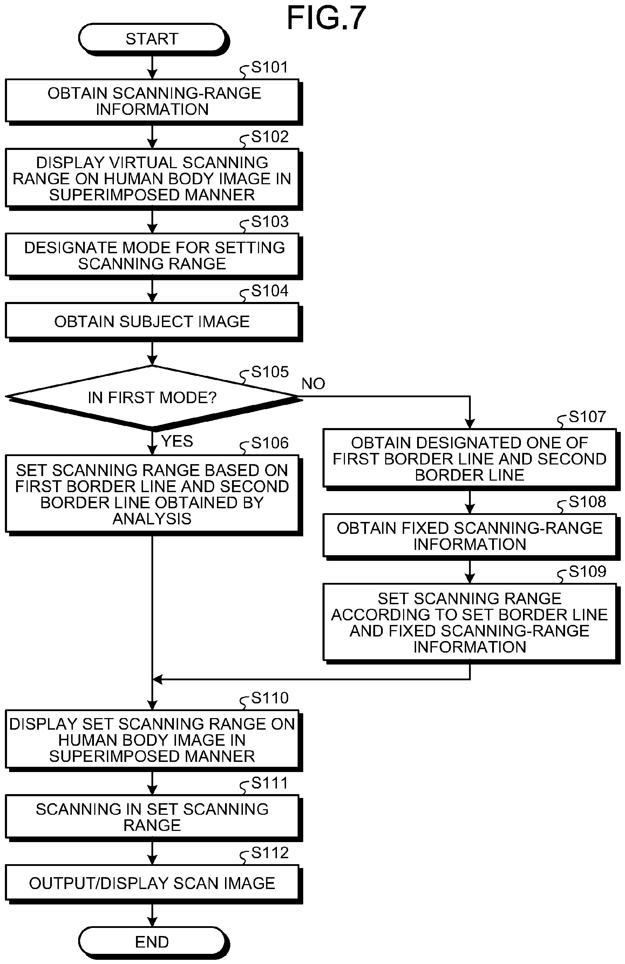

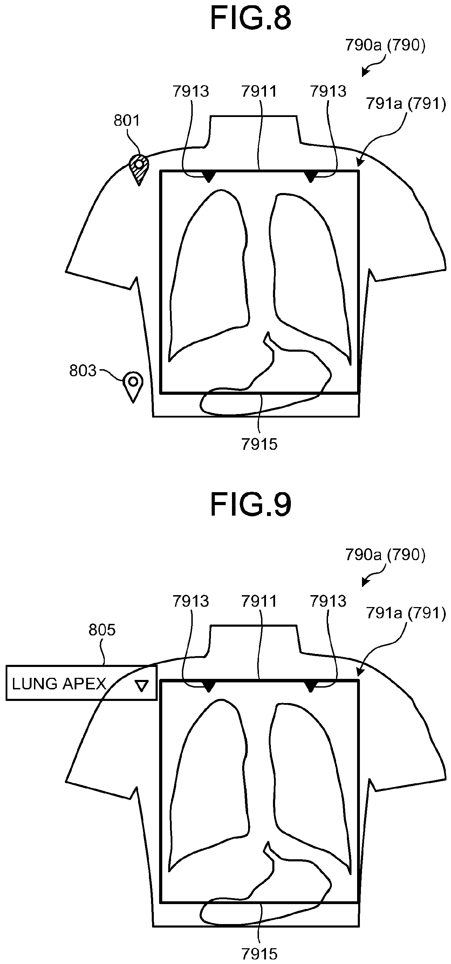

Next, setting of the scanning range 791 according to an embodiment will be described with reference to the drawings. FIG. 7 is a flowchart illustrating an exemplary procedure of scanning-range setting to be performed by the X-ray CT apparatus 1 according to an embodiment. FIG. 8 illustrates a first example of screen display related to setting of the automatic planning function for the scanning range, to be displayed on the display according to an embodiment.

Referring to FIG. 7, it is assumed that among the scan icons 513 displayed on the protocol display region 500, one representing a scan with the automatic scanning-range setting set be selected while the scan screen 130 or the protocol creation screen 170 is being displayed on the display 42, i.e., after the imaging protocol is selected.

The processing circuitry 44 (obtainer unit) obtains scanning-range information from, for example, the memory 41 (S101). The scanning-range information is information as to the scanning range of a selected scan. The memory 41 stores therein information containing imaging protocols or scans, regions to be imaged, and a margin in association with one another. The margin refers to information as to scan start position and end position with respect to landmarks in a region of interest, for example, setting a scan start position of an imaging protocol "detailed pulmonary field examination" at "1 cm above the landmark at the upper end of the right lung". The processing circuitry 44 (output unit) then displays a virtual scanning range (scanning range 791) on the human body image 790 including a front image 790a and a lateral image 790b representing a human body model in a superimposed manner on at least one of the scan screen 130 and the protocol creation screen 170 (S102), as illustrated in FIG. 8. The virtual scanning range simulates the scanning range in which the subject undergoes a selected scan to capture a medical image, and is set according to, for example, the scanning-range information and the region of interest by the selected scan. Alternatively, the virtual scanning range may be, for example, predefined for each region and stored in the memory 41.

The display of the virtual scanning range (scanning range 791) includes a first border line 7911 indicating a scan start position and a second border line 7915 indicating a scan end position. In other words, the first border line 7911 and the second border line 7915 are straight lines that define the virtual scanning range (scanning range 791). The display of the virtual scanning range (scanning range 791) further includes scan-direction indicators 7913.

The processing circuitry 44 further displays icons 801 and 803 for allowing inputs relative to the virtual scanning range on display. The icon 801 is for allowing inputs relative to the first border line 7911 indicative of the scan start position. For example, the processing circuitry 44 detects a selection of the scan start position from an output from the input interface 43 based on an input to the icon 801. The icon 803 is for allowing inputs relative to the second border line 7915 indicative of the scan end position. For example, the processing circuitry 44 detects a selection of the scan end position from an output from the input interface 43 based on an input to the icon 803.

The processing circuitry 44 (setting unit) designates a mode in which the scanning range of the selected scan is set (S103). Specifically, in response to a selection of only either of the scan start position and the scan end position, the processing circuitry 44 designates a second mode for the selected scan. The second mode is for deciding the scanning range 791 according to the selected scan start position or scan end position obtained by the automatic planning function and the fixed scanning-range information as to the selected scan. FIG. 8 depicts the icon 801 being in a selected state, by way of example. In the example of FIG. 8, thus, as to the selected scan, at the scan start position the automatic scanning-range planning function is set ON while at the scan end position the automatic scanning-range planning function is set OFF.

In response to no selection of the scan start position and the scan end position, the processing circuitry 44 designates a first mode for the selected scan. The first mode is for deciding the scanning range based on the scan start position and the scan end position set by the automatic planning function.

After setting the scan to which the automatic scanning-range planning function is applied, the processing circuitry 44 stores, as an imaging protocol preset, in the memory 41, the imaging protocol for which the scanning-range setting mode has been designated on the protocol creation screen 170, for example, and completes the procedure in FIG. 7. The processing circuitry 44 performs, for example, the automatic planning function for defining the scanning range in the designated mode for each of the scans displayed on the protocol display region 500 of the scan screen 130 to set the defined scanning range as the one in which a medical image of the subject P is obtained by the scan (S104 to S106). First, the processing circuitry 44 (obtainer unit) captures the subject to obtain a subject image (volume data) (S104). For example, the obtainer unit obtains image data of the subject. The processing circuitry 44 (setting unit) then sets the scanning range in the designated mode (S105 to S109). Specifically, the processing circuitry 44 determines as to the scan concerned whether or not the first mode is designated, i.e., which of the first mode and the second mode is designated (S105).

### First Mode

A description is now made on the automatic scanning-range planning for the scan with the first mode designated. After determining that the first mode is designated for the scan concerned (S105: Yes), the processing circuitry 44 sets the scanning range in the first mode (S106).

The processing circuitry 44 (analyzer unit) individually detects regions of the subject from the subject image. Specifically, the processing circuitry 44 detects a region such as an organ included in the subject image. For example, the processing circuitry 44 detects a region or a body part such as an organ from at least one of volume data of a localizer image and volume data of an image for use in diagnosis, with reference to anatomical landmarks. Herein, the term "anatomical landmarks" refers to points representing the features of a region such as a particular bone, organ, vessel, nerve, or lumen. The processing circuitry 44 thus detects, for example, a bone, an organ, a vessel, a nerve, or a lumen from the volume data by detecting anatomical landmarks such as a particular bone or organ. Further, the processing circuitry 44 can detect the positions of a head, a neck, a chest region, an abdominal region, and feet from the volume data by detecting the anatomical landmarks of the human body. In the present embodiment the term "region" signifies a bone, an organ, a vessel, a nerve, or a lumen and their respective positions, for example.

For example, the processing circuitry 44 (analyzer unit) extracts anatomical landmarks from voxel values included in the volume data of the localizer image or the volume data of the image for use in diagnosis. The processing circuitry 44 compares the positions of the landmarks extracted from the volume data and the three-dimensional positions of the anatomical landmarks according to, for example, textbook information, to find incorrect landmarks from among the extracted landmarks from the volume data. The processing circuitry 44 removes the incorrect landmarks from the extracted landmarks for optimization of the positions of the extracted landmarks. In this manner the processing circuitry 44 detects the respective regions of the subject from the volume data. As an example, the processing circuitry 44 first extracts anatomical landmarks from the volume data using a supervised machine learning algorithm. The supervised machine learning algorithm is constructed using multiple learning images with correct anatomical landmarks manually set, and is exemplified by a random decision forest.

The processing circuitry 44 (analyzer unit) compares the extracted landmarks and a model (human body model) representing a three-dimensional positional relationship among the anatomical landmarks in the human body for optimization of the extracted landmarks. This model is constructed using the above learning images and exemplified by a point distribution model. The model represents shapes of regions, their positional relationship, and/or points particular to the regions, which are defined based on the learning images with the correct anatomical landmarks manually set. In other words, the human body model is a model simulating the anatomical landmarks in the human body and may be referred to as a virtual human body model. The virtual human body model may represent a three-dimensional positional relationship among the anatomical landmarks in the human body. Thus, the processing circuitry 44 compares such the above model and the extracted landmarks to thereby remove the incorrect landmarks for optimization of the landmarks.

As described above, the processing circuitry 44 (analyzer unit) can identify the types of the landmarks and the positions thereof in the volume data of the localizer image or of the image for use in diagnosis, to be able to detect the respective regions as organs based on such information. For example, the processing circuitry 44 detects the position of a region of interest based on information as to an anatomical positional relationship between the region of interest and regions around the region of interest. As an example, in the case of the lungs being the region of interest, the processing circuitry 44 obtains coordinate information associated with identification codes for the features of the lungs as well as coordinate information associated with identification codes for the features of the regions surrounding the lungs, such as costal bones or clavicle bones, the heart, and the diaphragm. The processing circuitry 44 then extracts a pulmonary region from the volume data based on the obtained coordinate information and the information as to the anatomical positional relationship between the lungs and the regions surrounding the lungs.

The processing circuitry 44 (analyzer unit) obtains the scan start position and the scan end position according to the region of interest extracted through the analysis of the subject image and on the scanning-range information obtained from the memory 41, as described above. The processing circuitry 44 (setting unit) defines the scanning range based on the scan start position and the scan end position (first border line and second border line).

Thus, in response to the border line designation, the processing circuitry 44 serving as an analyzer unit performs the analysis of the image data with reference to the border line set on the human body image and the human body model, to determine at least one border line of the scanning range for a CT scan of a region to be imaged of the subject. Specifically, the processing circuitry 44 extracts anatomical landmarks from the image data and compare the extracted anatomical landmarks and the three-dimensional positional relationship among the anatomical landmarks in the human body model, to detect a region to be imaged from the image data. The processing circuitry 44 determines at least one border line of the scanning range for a CT scan of the region to be imaged of the subject, based on the detected region to be imaged and the at least one border line of the scanning range on the human body image. The processing circuitry 44 implementing a setting unit sets, with reference to the determined border line, the scanning range to be displayed together with the image data on the display 42.

### Second Mode

Next, the automatic scanning-range planning for a scan with the second mode designated will be described.

After determining that the second mode is designated for the scan concerned (S105: No), the processing circuitry 44 sets the scanning range in the second mode (S107 to S109). The processing circuitry 44 (analyzer unit) obtains one of the scan start position and the scan end position at which the automatic planning is validated, based on the region of interest extracted through the analysis of the subject image and on the scanning-range information obtained from the memory 41, as described above. The processing circuitry 44 (analyzer unit) obtains a designated one of the scan start position and the scan end position (S107). The processing circuitry 44 (analyzer unit) further obtains fixed scanning-range information as to the selected scan from, for example, the memory 41, in addition to the scanning-range information obtained in S101 (S108). The memory 41 stores therein information on the Z-directional lengths (fixed scanning-range information) including the imaging protocols or scans, regions to be imaged, and a margin in association with one another, in addition to the scanning-range information.
In an embodiment, the user of the CT or other medical professional can determine the fixed range which is stored in the memory (41). When a doctor reviews multiple CT images acquired from multiple patients, sequentially, it is beneficial if the size/range is fixed. It can be difficult to review multiple images if the size of the image changes by patient. The fixed scanning-range information may be obtained in S101. The processing circuitry 44 (setting unit) defines the scanning range according to either of the scan start position and the scan end position and the fixed scanning-range information obtained from the memory 41 (S109).

The processing circuitry 44 (output unit) outputs the scanning range 791 of each of the scans, set in the first mode or the second mode as designated, for display on the display 42 (S110). Specifically, the processing circuitry 44 displays, in a superimposed manner, the scanning range 791 of the currently selected scan on the human body image 790 including the front image 790a and the lateral image 790b.

The processing circuitry 44 (imaging unit) performs a CT scan of the subject P in the scanning range set in the first mode or the second mode (5111). The processing circuitry 44 output unit) then outputs the image resulting from the scan (reconstructed image) to the display 42 for display on the display 42 (S112). As an example, the processing circuitry 44 displays the image resulting from the scan on the imaging information display region 750 of the scan screen 130. The processing circuitry 44 displays the image resulting from the scan on the scan screen 130 of FIG. 6, for example, in a display area on the right side of the human body image 790, in place of the human body image 790.

As such, in the medical image diagnostic apparatus according to an embodiment the processing circuitry 44 can limit objects to which the automatic planning function is applied, in accordance with an output from the input interface 43 based on a user input. Specifically, the medical image diagnostic apparatus according to an embodiment includes a means to switch ON and OFF the automatic planning function with respect to either of the scan start position and the scan end position.

Owing to the features as above, to apply a different scanning range from the general scanning range in compliance with the policies of the medical facility that uses the medical image diagnostic apparatus, for example, at the time of a follow-up examination, the medical image diagnostic apparatus can abate the trouble with manually correcting the scanning range of the scan concerned after the automatic setting. Alternatively, with respect to any scan, the medical image diagnostic apparatus can turn off the automatic scanning-range setting and then turn it on again, or apply the automatic setting again after switching the scan start position and the scan end position as the object to be applied with the automatic setting. In this manner the medical image diagnostic apparatus according to an embodiment can abate the trouble with setting the conditions for the individual scans, thereby improving the throughput of the image diagnosis using the medical image diagnostic apparatus.

The scanning range 791 including the virtual scanning range can be adjusted on the display by an operator's input. Suppose a situation that the input interface 43 include, for example, a mouse and the scanning range be adjusted with the mouse. The scanning ranges 791a and 791b are generally adjusted in size in the Z and X-directions on the front image 790a while adjusted in the Z and Y-directions on the lateral image 790b. Meanwhile, to adjust the scan region in position, it is convenient to use a frame representing the scan region as movable in any direction. While the scan region frame is being selected and a first button (for example, right click) of the mouse is being pressed, the processing circuitry 44 moves the frame in any direction in accordance with the moving direction of the mouse. While a second button (for example, left click) of the mouse is being pressed, the processing circuitry 44 control the frame to be moved only vertically or horizontally on the display screen. The processing circuitry 44 performs such control of the frame by extracting vertical or horizontal components from the moving direction of the mouse and moving the frame in accordance with the components.

The vertical or horizontal direction may be selected, for example, according to a direction in which the mouse is initially moved in a predetermined amount or more after a start of the manipulation of the second button. For example, while the second button is being manipulated, the vertical and horizontal mouse movements are separately accumulated to select, as the moving direction, the direction of which the accumulated amount exceeds a predetermined threshold earlier. As an example, upon a selection of the vertical direction, moving the frame according to the mouse movement may be restricted, e.g., not allowed, during the accumulation period. Upon a selection of a first (e.g., vertical) direction, the processing circuitry 44 moves the frame in the first direction according to the moving direction of the mouse while restricting the frame movement in a second (e.g., horizontal) direction. Upon a selection of the second direction, the processing circuitry 44 moves the frame in the second direction according to the moving direction of the mouse while restricting the frame movement in the first direction. Upon completion of the manipulation of the mouse, the processing circuitry 44 lifts the restrictions on the moving direction of the frame. By such a control, the processing circuitry 44 can efficiently adjust the scanning ranges 791a and 791b.

### Second Embodiment

The first embodiment has described an example of applying the automatic planning function to the scan start position or the scan end position, however, it is not limited to such an example.

In the medical image diagnostic apparatus according to another embodiment, the processing circuitry 44 displays an icon similar to the icon 801 or the icon 803 in a center (e.g., a position between the icons 801, 803 in FIG. 8) of the virtual scanning range. This icon is for allowing inputs to the virtual scanning range and a scan center position on display.

For example, the processing circuitry 44 detects a selection of the scan center position from an output from the input interface 43 based on an operational input to the icon. Responding to the selection of the scan center position, the processing circuitry 44 designates the second mode for the selected scan. The second mode is for defining the scan center position by the automatic planning function and defining the scanning range 791 according to the fixed scanning-range information as to the selected scan and either of the scan start position and the scan end position depending on the scan center position.

Additionally, the processing circuitry 44 can display the scan center position in another display form. FIG. 9 illustrates a second example of screen display related to setting of automatic planning function for the scanning range, to be displayed on the display according to an embodiment. The processing circuitry 44 displays an icon 805 that allows inputs to the virtual scanning range on display. The icon 805 allows the operator to select a region of interest from a pull-down menu of regions to be imaged. The icon 805 also allows inputs to the scan center position on display. For example, the processing circuitry 44 detects a selection of the scan center position relative to the selected region from an output from the input interface 43 based on an input to the icon 805.

As such, in the medical image diagnostic apparatus according to the second embodiment, the processing circuitry 44 can limit objects to which the automatic planning function is applied, in accordance with an output from the input interface 43 based on a user input. Specifically, the medical image diagnostic apparatus according to the second embodiment includes a means to switch ON and OFF the automatic planning function with respect to the scan center position. Owing to such features, the medical image diagnostic apparatus of the second embodiment can attain the effects similar or same as the above embodiments in a scan mode (e.g., dynamic volume scan (non-helical)) with no couch movement involved, such as a conventional scan or a volume scan.

### Third Embodiment

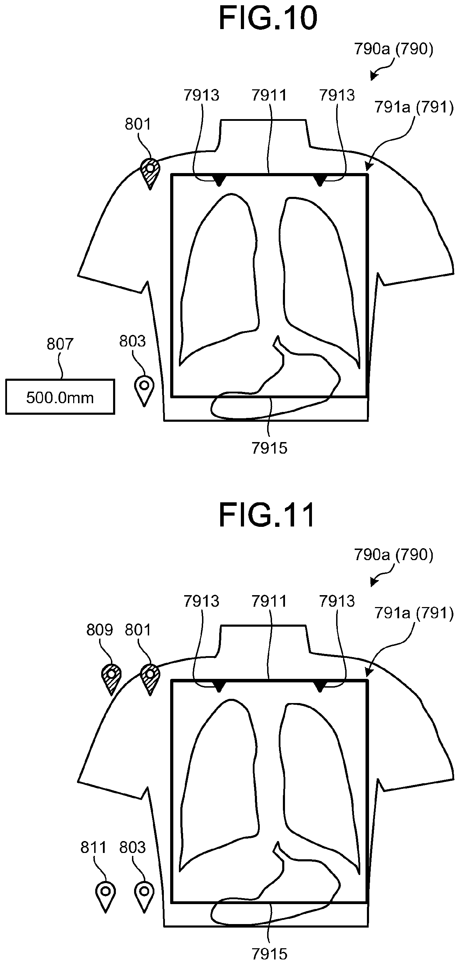

The medical image diagnostic apparatus of the first embodiment may receive the fixed scanning-range information by an operator's input. FIG. 10 illustrates a third example of screen display related to setting of automatic planning function for the scanning range, to be displayed on the display according to an embodiment.

For example, the processing circuitry 44 displays an entry box 807 for a Z-directional scan length, responding to a selection of only either the scan start position or the scan end position. According to an output from the input interface 43 based on an operator's input, the processing circuitry 44 obtains an input scan length as fixed scanning-range information as to the scan concerned and sets the scanning range in the second mode. As illustrated in FIG. 9, for example, the processing circuitry 44 may display a screen including the icon 805 for allowing a selection of a region to be imaged. Responding to an output from the input interface 43 based on an operator's input, the processing circuitry 44 may obtain, as the fixed scanning-range information, a Z-directional scan length which is stored in the memory 41 in association with the selected region.

Owing to such features, the medical image diagnostic apparatus can make the scanning range defined by the automatic planning function closer to an operator's desirable scanning range, thereby abating his or her trouble with manually correcting the scanning range and improving the throughput of the image diagnosis using the medical image diagnostic apparatus.

### Fourth Embodiment

The medical image diagnostic apparatus of the first embodiment may be configured to allow preferential setting. FIG. 11 illustrates a fourth example of screen display related to setting of automatic planning function for the scanning range, to be displayed on the display according to an embodiment.

The processing circuitry 44 additionally displays icons 809 and 811 for allowing inputs to the virtual scanning range on display. The icons 809 and 811 are for allowing inputs to designate either the scan start position or the scan end position as being preferentially maintained, at the time of setting the scanning range to suffice a designated minimum scanning range by the automatic planning function. From an output from the input interface 43 based on an operator's input to the icon 809, for example, the processing circuitry 44 detects a selection of the scan start position as preferentially set. From an output from the input interface 43 based on an operator's input to the icon 811, the processing circuitry 44 detects a selection of the scan end position as preferentially set.

The processing circuitry 44 may not concurrently display the icons 801, 803, 809, and 811 but may switch between an object setting screen including the icons 801 and 803 and a preferential setting screen including the icons 809 and 811 on the display.

In addition, at the time of preferential setting, the processing circuitry 44 can obtain, for example, a designated minimum scanning range according to an input similar or same as the one for the scanning range adjustment as above. Alternatively, the minimum scanning range may be stored in the memory 41 as the fixed scanning-range information. The processing circuitry 44 can also obtain the designated minimum scanning range from previous examination information as to the same subject P.

For example, when the scanning range set in the first mode or the second mode is less than the designated minimum scanning range, the processing circuitry 44 defines the scanning range by extending a selected one of the scan start position and the scan end position while maintaining the other.

By means of an icon similar to the icons 809 and 811, the scan center position may be preferentially set. In this case the processing circuitry 44 defines the scanning range sufficing the designated minimum scanning range, by extending at least one of the scan start position and the scan end position while maintaining the scan center position.

As such, in the medical image diagnostic apparatus according to the fourth embodiment the processing circuitry 44 can set an object to be preferentially maintained at the time of setting the scanning range of the scan using the automatic planning function, in accordance with an output from the input interface 43 based on a user input. Specifically, the medical image diagnostic apparatus according to the present embodiment includes a means to switch ON and OFF the preferential setting for the scan with the automatic planning function applied. Owing to such features, the medical image diagnostic apparatus can make the scanning range set by the automatic planning function closer to an operator's desired scanning range, thereby abating his or her trouble with manually correcting the scanning range and improving the throughput of image diagnosis using the medical image diagnostic apparatus.

### Fifth Embodiment

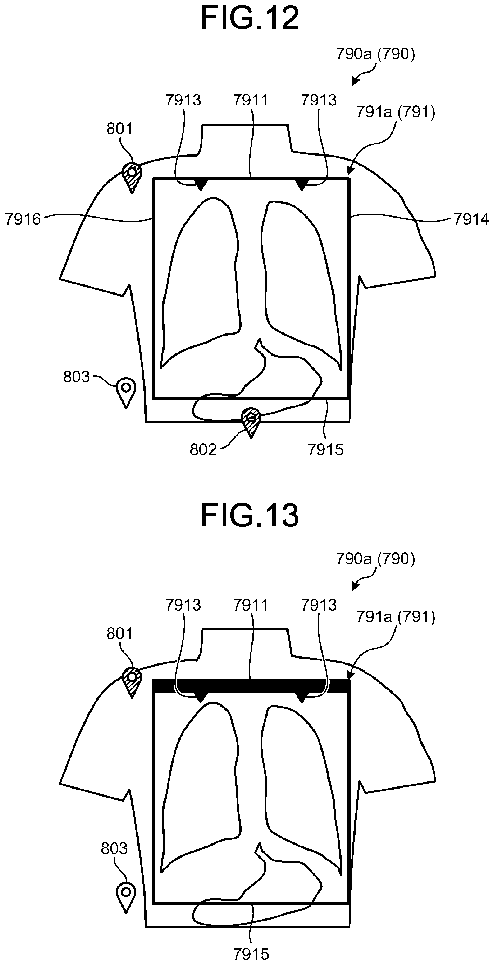

The above embodiments have described examples of setting application or non-application of the automatic planning function with respect to the scan start position, the scan end position, and the scan center position. Likewise, the medical image diagnostic apparatus can set application or non-application of the automatic planning function with respect to an FOV in the scanning range. FIG. 12 illustrates a fifth example of screen display related to setting of automatic planning function for the scanning range, to be displayed on the display according to an embodiment.

According to the medical image diagnostic apparatus of the present embodiment, the first border line 7911, the second border line 7915, border line 7914 and border line 7916 define an FOV size in the scanning range.

As an example, the processing circuitry 44 additionally displays an icon 802 that allows inputs to the virtual scanning range on display. The icon 802 allows inputs to designate application of the automatic planning function with respect to a FOV size in the scanning range. For example, the processing circuitry 44 detects a selection of the FOV size from an output from the input interface 43 based on an input to the icon 802. At the same time the processing circuitry 44 may acquire the center position of the FOV as the fixed scanning-range information. The FOV center position may be obtained based on an operator's input or previous examination information as to the same subject P.

As another example, the processing circuitry 44 additionally displays the icon 803 that allows inputs to the virtual scanning range on display. The icon 803 allows inputs to designate application of the automatic planning function with respect to the FOV center position in the scanning range. For example, the processing circuitry 44 detects a selection of the FOV center position from an output from the input interface 43 based on an input to the icon 803.

In this case the processing circuitry 44 obtains a designated minimum FOV size to be ensured, based on an operator's input or previous examination information as to the same subject P.

Alternatively, the processing circuitry 44 may obtain a designated maximum magnification factor of the FOV based on an operator's input or previous examination information as to the same subject P.

When the scanning range set in the first mode or the second mode is less than at least one of the minimum FOV size and the FOV maximum magnification factor as designated, the processing circuitry 44 defines the scanning range by expanding the FOV size while maintaining the FOV center position. Either the minimum FOV size or the FOV maximum magnification factor may not be used depending on an embodiment.

As such, in the medical image diagnostic apparatus according to the fifth embodiment, the processing circuitry 44 can limit objects to which the automatic planning function is applied, in accordance with an output from the input interface 43 based on a user input. Specifically, the medical image diagnostic apparatus according to the fifth embodiment includes a means to switch ON and OFF the automatic planning function with respect to at least one of the FOV center position and the FOV size. Owing to such features, the medical image diagnostic apparatus can maintain the pixel size, i.e., magnification factor, which makes it possible to facilitate the comparison between the images resulting from different examinations such as follow-up examination, between the images of different patients, or between the images of the same patient at different instances. This can improve the throughput of the image diagnosis using the medical image diagnostic apparatus.

### Sixth Embodiment

According to any of the above embodiments, the operator may be notified of the details of the scanning range adjustment by the automatic planning function. FIG. 13 illustrates a sixth example of screen display related to setting of automatic planning function for the scanning range, to be displayed on the display according to an embodiment.

During a preparation of an imaging protocol or adjustment of an imaging protocol before start of a scan, a localizer scan may be performed in relation to a scan with the automatic planning function set ON. In such a case the processing circuitry 44 displays, in a highlighted manner, items automatically adjusted (e.g., determined border line) based on the resultant localizer image by the automatic planning function. FIG. 13 depicts the scan start position when automatically adjusted, by way of example. In this case, as illustrated in FIG. 13, the processing circuitry 44 displays the first border line 7911 indicating the scan start position and the scan-direction indicators 7913 in a highlighted manner, to notify the operator of the adjusted items. The scan-direction indicators 7913 may not be highlighted. Alternatively, the operator may be notified of the adjusted items by repetitions of a highlighted display and a non-highlighted display of the items in an alternative manner. The operator may be notified of the adjusted items by an audio output from a speaker.

Such features make it possible for the operator to easily recognize the automatically adjusted items after selecting a protocol with the automatic scan-planning function set valid to perform a localizer scan. Thereby, the operator can easily determine whether the scan concerned has been set as he or she has intended.

### Seventh Embodiment

The above embodiments have described examples of displaying the virtual scanning range and the set scanning range 791 on the human body image 790 including the human body model in a superimposed manner, however, they are not limited to such examples. The virtual scanning range and the set scanning range 791 may be displayed on another image in a superimposed manner.

For example, before a scanogram is obtained, the human body model may be used while after a scanogram is obtained, a subject image resulting from the scanogram, i.e., a low-dose CT scan may be used. In the case of a scanogram such as a helical scan or a conventional scan (dynamic volume scan) with no couch movement involved, the processing circuitry 44 may generate front image data and lateral image data from a three-dimensional image acquired by the scan concerned, to display the front image 790a and the lateral image 790b on the display 42.

Alternatively, the medical image diagnostic apparatus according to an embodiment may include an optical camera capable of capturing the subject P lying on the table top 33 of the couch 30. In such a case the processing circuitry 44 may display the virtual scanning range and the scanning range 791 on the image captured by the optical camera in a superimposed manner. In addition, the processing circuitry 44 may obtain an image of the subject P lying on the table top 33 of the couch 30 captured by an optical camera placed outside the medical image diagnostic apparatus, such as a surveillance camera installed in the examination room where the medical image diagnostic apparatus is located.

The processing circuitry 44 may perform the automatic scanning-range planning based on the image captured by the optical camera, in addition to the subject image obtained from the scanogram.

The term "processor" used herein signifies, for example, circuitry such as a CPU, a graphical processing unit (GPU), an application specific integrated circuit (ASIC), and a programmable logic device (PLD). The PLD includes a simple programmable logic device (SPLD), a complex programmable logic device (CPLD), and a field programmable gate array (FPGA). Such a processor reads and executes programs from the memory circuitry to implement the functions. The memory circuitry storing the programs is a non-transitory computerreadable storage medium. In place of being stored in the memory circuitry, the programs may be directly embedded in the circuitry of the processor. In such a case the processor reads and executes programs from its own circuitry to implement the functions. Further, in place of executing the programs, the functions corresponding to the programs may be implemented by a combination of logic circuits. According to the above embodiments, the processors may not be individually configured as a single circuit. A single processor may be configured of a combination of independent circuits to implement the functions. The elements illustrated in FIG. 1 may be integrated into a single processor to implement the functions.

According to at least one of the embodiments as above, it is possible to improve the throughput of the image diagnosis using the medical image diagnostic apparatus.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical image diagnosis apparatus (1), comprising:
an analyzer unit (45) configured to obtain a first border line (7911) and a second border line (7915) that define a scanning range of a scan of a subject to obtain a medical image of the subject, by analyzing a subject image obtained by capturing the subject;
an obtainer unit (45) configured to obtain fixed scanning-range information for imaging the subject;
a setting unit (45) configured to set the scanning range based on the fixed scanning-range information and either of the first border line (7911) and the second border line (7915) obtained by the analyzer unit (45); and
an output unit (48) configured to output the scanning range set by the setting unit for display on a monitor (42).

2. The medical image diagnosis apparatus (1) to claim 1, wherein
the obtainer unit (45) is further configured to obtain the subject image,
the output unit (48) is further configured to set, per region to be imaged, at least one border line of a scanning range on a human body image representing a human body model, and display, on the monitor (42), a graphical user interface that allows a designation or a non-designation of a border line of the scanning range based on analysis of the subject image;
the analyzer unit (45) is further configured to, in response to the designation, determine at least one border line of the scanning range for a scan of a region to be imaged of the subject by performing the analysis of the subject image with reference to the border line set on the human body image and the human body model; and
the output unit (48) is further configured to display the scanning range based on the determined border line together with the subject image on the monitor (42).

3. The medical image diagnosis apparatus (1) according to claim 2, wherein
the human body model represents a three-dimensional positional relationship among anatomical landmarks in a human body,
the analyzer unit (45) is further configured to:
extract anatomical landmarks from the subject image,
compare the extracted anatomical landmarks and the three-dimensional positional relationship among anatomical landmarks in the human body model, to detect a region to be imaged in the subject image, and
determine at least one border line of the scanning range for a scan of the region to be imaged of the subject, based on the detected region to be imaged and the at least one border line of the scanning range on the human body image, and
the setting unit (45) is further configured to set, with reference to the determined border line, the scanning range to be displayed together with the subject image on the monitor (42).

4. The medical image diagnosis apparatus (1) according to claim 3, wherein
the at least one border line set on the human body image includes:
the first border line representing one of a start position and an end position of a scan in the scanning range on the human body image, and
the second border line representing the other of the start position and the end position,
the apparatus (1) further comprises an input unit (43) configured to receive an input to designate only one of the first border line and the second border line, and
the setting unit (45) is further configured to:
when the set scanning range to be displayed together with the subject image on the monitor (42) is less than a minimum scanning range to be ensured as the scanning range designated by the input, set the scanning range to suffice the minimum scanning range by extending one of the start position and the end position and maintaining the other.

5. The medical image diagnosis apparatus (1) according to claim 3, wherein
the at least one border line set on the human body image includes:
the first border line representing one of a start position and an end position of a scan in the scanning range on the human body image, and
the second border line representing the other of the start position and the end position,
the apparatus (1) further comprises an input unit (43) configured to receive an input to designate either of the first border line and the second border line as being preferentially maintained, in setting the scanning range on the human body image, and
the setting unit (45) is further configured to:
set the scanning range to be displayed together with the subject image on the monitor (42) such that the scanning range suffices a minimum scanning range to be ensured as the scanning range designated by the input, by maintaining one of the start position and the end position designated by the input and extending the other.

6. The medical image diagnosis apparatus (1) according to claim 3, wherein
the setting unit (45) is further configured to:
designate a minimum scanning range to be ensured as the scanning range on the human body image, based on previous examination information of the subject, and
when the set scanning range to be displayed together with the subject image on the monitor (42) is less than the minimum scanning range, set the scanning range to suffice the minimum scanning range by extending one of a start position and the end position of a scan in the scanning range on the human body image and maintaining the other.

7. The medical image diagnosis apparatus (1) according to claim 3, wherein
the at least one border line set on the human body image includes:
the first border line representing one of a start position and an end position of a scan in the scanning range on the human body image, and
the second border line representing the other of the start position and the end position,
the apparatus (1) further comprises an input unit (43) configured to receive an input to designate a center position of the scan in the scanning range on the human body image,
the obtainer unit (45) is further configured to obtain fixed scanning-range information as to a scan mode with no couch movement, and
the setting unit (45) is further configured to set the scanning range to be displayed together with the subject image on the monitor (42), based on the fixed scanning-range information and one of the first border line and the second border line set according to the center position of the scan.

8. The medical image diagnosis apparatus (1) according to claim 7, wherein
the setting unit (45) is further configured to:
when the set scanning range to be displayed together with the subject image on the monitor (42) is less than a minimum scanning range to be ensured as the scanning range designated by the input, set the scanning range to suffice the minimum scanning range by extending at least one of the first border line and the second border line and maintaining the center position of the scan.

9. The medical image diagnosis apparatus (1) according to claim 7, wherein
the setting unit (45) is further configured to:
designate a minimum scanning range to be ensured as the scanning range on the human body image, based on previous examination information of the subject, and
when the scanning range to be displayed together with the subject image on the monitor (42) is less than the minimum scanning range, set the scanning range to suffice the minimum scanning range by extending at least one of the first border line and the second border line and maintaining the center position of the scan.

10. The medical image diagnosis apparatus (1) according to claim 8 or claim9, wherein
the input unit (43) is further configured to receive an input to designate the center position of the scan as being preferentially maintained, in setting the scanning range, and
the setting unit (45) is further configured to set the scanning range to suffice the minimum scanning range by extending at least one of the first border line and the second border line and maintaining the center position of the scan.

11. The medical image diagnosis apparatus (1) according to claim 3, wherein
the at least one border line set on the human body image includes:
the first border line representing one of a start position and an end position of a scan in the scanning range on the human body image, and
the second border line representing the other of
the start position and the end position,
the first border line and the second border line define a field of view (FOV) size in the scanning range,
the apparatus (1) further comprises an input unit (43) configured to receive an input to designate an FOV size, and
the setting unit (45) is further configured to set the scanning range to be displayed together with the subject image on the monitor (42), in response to a receipt of the input.

12. The medical image diagnosis apparatus (1) according to claim 10, wherein
the setting unit (45) is further configured to:
when the scanning range to be displayed together with the subject image on the monitor (42) is less than a minimum FOV size to be ensured as the FOV size designated by the input, set the scanning range to suffice the minimum FOV size by extending at least one of the first border line and the second border line and maintaining the center position of the FOV.

13. The medical image diagnosis apparatus (1) according to claim 10, wherein
the input unit (43) is further configured to receive an input to designate a minimum FOV size to be ensured as the FOV size designated by the input, based on previous examination information of the subject, and
the setting unit (45) is further configured to:
when the set scanning range to be displayed together with the subject image on the monitor (42) is less than the minimum FOV size, set the scanning range to suffice the minimum FOV size by extending at least one of the first border line and the second border line and maintaining the center position of the FOV.

14. The medical image diagnosis apparatus (1) according to any one of claims 3, 4, 6, 9 to 11, and 13, further comprising:
an optical camera that captures the subject, wherein
the output unit (48) is further configured to output a virtual scanning range simulating the scanning range, to display, on the monitor (42), the virtual scanning range on an image of the subject captured by the optical camera in a superimposed manner, and
the input is directed to the scanning range on display.

15. A scanning-range setting method, comprising:
obtaining a first border line and a second border line that define a scanning range of a scan of a subject to obtain a medical image of the subject, by analyzing a subject image obtained by capturing the subject;
obtaining fixed scanning-range information for imaging the subject;
setting the scanning range based on the fixed scanning-range information and either of the first border line and the second border line obtained by the analyzing; and
outputting the scanning range set by the setting for display on a monitor (42).
